(19) Europäisches Patentamt
European Patent Office
Office européen des brevets

(11) EP 2 809 329 B1

(12) EUROPEAN PATENT SPECIFICATION

(45) Date of publication and mention of the grant of the patent:
**01.08.2018 Bulletin 2018/31**

(21) Application number: **13714546.2**

(22) Date of filing: **30.01.2013**

(51) Int Cl.:
***A61K 31/718*** (2006.01) ***A61P 35/00*** (2006.01)
***A61K 45/06*** (2006.01) ***A61K 31/337*** (2006.01)
***A61K 31/4045*** (2006.01) ***A61K 31/7068*** (2006.01)

(86) International application number:
**PCT/EP2013/000276**

(87) International publication number:
**WO 2013/113496 (08.08.2013 Gazette 2013/32)**

(54) **HYDROXYALKYL STARCH IN COMBINATION WITH CYTOSTATICA FOR THE TREATMENT OF CANCERS BY REDUCTION OF TUMOR GROWTH RATES**

HYDROXYALKYLSTÄRKE IN KOMBINATION MIT ZYTOSTATIKA ZUR BEHANDLUNG VON KREBS DURCH REDUKTION VON TUMORWACHSTUMSRATEN

AMIDON HYDROXY-ALKYLÉ EN COMBINAISON AVEC DES CYTOSTATIQUES POUR LE TRAITEMENT DE CANCERS PAR RÉDUCTION DE TAUX DE CROISSANCE TUMORALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **30.01.2012 EP 12153068**
**30.01.2012 US 201261592017 P**

(43) Date of publication of application:
**10.12.2014 Bulletin 2014/50**

(73) Proprietor: **Fresenius Kabi Deutschland GmbH**
**61352 Bad Homburg (DE)**

(72) Inventors:
- **WESTPHAL, Martin**
  **61350 Bad Homburg (DE)**
- **BAASNER, Silke**
  **61137 Schöneck (DE)**

(74) Representative: **Fresenius Kabi Deutschland GmbH**
**Patent Department**
**Borkenberg 14**
**61440 Oberursel (DE)**

(56) References cited:
**DE-A1- 4 023 788 US-B1- 6 207 654**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 809 329 B1

## Description

Cancer, tumor-associated diseases and neoplastic disease states are serious and often life-threatening conditions. These diseases, which are characterized by uncontrolled cell proliferation, also called cell proliferating diseases, are also a focal point of many research projects, devoted to identifying new active therapeutic ingredients which prove to be effective in the treatment of these diseases. Such active ingredients prolong the life expectancy of the patient, inhibit the rapidly progressing cell growth associated with the neoplasm, or bring about regression of the neoplasm, or improve the quality of life.

Hydroxyalkyl starches (HAS) are polymers which are derived from natural base materials and are modified. HAS are prepared from amylopectin-rich starches. The parent starch may be branched or unbranched, or may consist of a mixture of both. Hydroxyethyl starches are based almost exclusively on amylopectin, in other words on branched chains of glucose molecules.

The medical use of hydroxyalkyl starches, and more particularly of hydroxyethyl starch, is known. It is used in particular in volume therapy as a plasma substitute, and also in clinical haemodialysis (Sommermeyer et al., 1987, Krankenhauspharmazie, 8(8): 271-278; Weidler et al., 1991, Arzneimittelforschung/Drug Research, 41: 494-498). Intravenous administration of a hydroxyethyl starch solution, which allows the erythrocytes, (red blood corpuscles), to continue transporting oxygen through the body, can be used, for example, in order to prevent a state of shock following severe blood loss caused by trauma or by surgery.

Additionally it has been proposed to use hydroxyethyl starch (HES) to introduce active pharmaceutical ingredients into the peritoneum. In some treatment regimens of peritoneal carcinomatosis the cytotoxic or cytostatic drugs are applied locally. Here it has been shown that the local use of solutions containing HES results in higher retention times of the cytotoxic or cytostatic drug in the peritoneum, compared to the use of dialysis solutions free of osmotically active colloids (Mohamed et al (2003) European Journal of Surgical Oncology vol 29, p 261-265).

DE4023788 (Schumann) describes the use of hydroxyethyl starch for the treatment of damage of the inner ear in a treatment called hyperbaric oxygen therapy applied to patient in an oxygen pressure chamber. It is suggested to treat a number of diseases including cancer, but the disclosure fails to demonstrate or discuss any therapeutic effect.

US 6,207,654 (Zikria) teaches that hydroxyethyl starch may be used to improve a cancer treatment based on administration of interleukins, by reducing the side effects of interleukin 2 (IL-2). It is explained that the polysaccharide molecules act as endothelial membrane stabilizers. Due to their biophysical/biochemical properties, they seem to prevent leakage of serum protein from capillary endothelial junctions by sealing these and thereby stabilizing the capillary membranes.

It has also been suggested to use HES as an absorbable barrier, as an anti-adhesion agent in injured body cavities (WO 96/40108).

Many of the usual cytotoxic or cytostatic ingredients, together referred to as cytostatica these being active ingredients which inhibit cell growth, that are used in present-day cancer therapy have only low water solubility. This presents problems for their administration. The low water-solubility must typically be overcome by means of complex formulation techniques, such as by the addition of various excipients, which in general entail toxic side-effects. One possible solution proposed has been the coupling of cytostatica to macromolecular carriers, such as hydroxyalkylated starch, for example, in order to enable the administration of so called polymeric prodrugs.

Besides the enhancement of the water solubility of the drug, prodrugs have been proposed to provide an advantageous targeting and/or an enhancement of the stability of the therapeutic agent. Further, such prodrugs were suggested to prolong the circulation lifetime, to provide an extended duration of activity, or to achieve a reduction of side effects and drug toxicity. Thus, besides the preparation of prodrugs of water insoluble cytotoxic or cytostatic agents, providing prodrugs of water soluble cytotoxic or cytostatic agents is also of high interest in order to modify the onset and/or duration of action of the cytotoxic agent *in vivo.*

WO 03/074088 describes hydroxyalkyl starch conjugates with, for example, cytostatica such as daunorubicin, wherein the cytostatica are usually directly coupled via an amine group to the hydroxyalkyl starch yielding in 1:1 conjugates. However no use of these conjugates *in vivo* was shown therein.

Currently, the routine cancer treatment relies on three treatment options, surgery, radiation therapy and drug therapy, commonly referred to as chemotherapy. Chemotherapy involves the administration of drugs, which are designed to kill highly proliferating cells, such as cancer cells, or tumor cells, or at least to stop them from proliferating any further. They are commonly referred to as cytostatica. These drugs however are not selective in killing only the cancer cells. Hence this type of treatment is associated with severe side effects for the patient. A non-comprehensive list of side effects comprises anemia, nausea, vomiting, appetite changes, diarrhea, constipation, fatigue, pain, hair loss, bleeding, swelling,

DE4023788 (Schumann) describes the use of hydroxyethyl starch for the treatment of damage of the inner ear in a treatment called hyperbaric oxygen therapy applied to patient in an oxygen pressure chamber. It is suggested to treat a number of diseases including cancer, but the disclosure fails to demonstrate or discuss any therapeutic effect.

US 6,207,654 (Zikria) teaches that hydroxyethyl starch may be used to improve a cancer treatment based on administration of interleukins, by reducing the side effects of interleukin 2 (IL-2). It is explained that the polysaccharide molecules act as endothelial membrane stabilizers. Due to their biophysical/biochemical properties, they seem to prevent leakage of serum protein from capillary endothelial junctions by sealing these and thereby stabilizing the capillary membranes.

increased susceptibility for infection, reduced memory, nerve changes, mouth and throat changes, sexual and fertility changes, skin and nail, urination problems. These side effects can be so severe, that the treatment with cytostatica has to be stopped due to the high toxicity, in order to keep the patient alive. However, during these phases of recovery, wherein the patient may regain some general health, the tumor often also recovers and starts to grow again. The problem of the high toxicity of cytostatic drugs used to treat cancer patients is well known. Hence there is a need in the art for a treatment option for cancer patients, which inhibits progression of cancer while not stressing or impairing the subject in need of treatment any further.

An ideal cancer treatment would target the tumor growth and the tumor cell proliferation selectively. Healthy cell proliferation at a normal and controlled rate would be unaffected.

It is one aspect of the current invention to provide for a treatment option that allows treating a cancer patient with a hydroxyalkylated starch, that is effective in reducing tumor growth rate, and at the same time showing no or significantly less toxic side effects, when given in combination with a treatment based on the standard of care drug, which usually is a compound selected from the group consisting of cytostatica, biologicals with anti-cancer activity and hormons with anti-cancer activity.

In another aspect of the invention the combination therapy is defined as treating the patient suffering from a tumor with the substances according to the invention prior to the beginning of the treatment regimen with a compound selected from the group consisting of cytostatica, biologicals with anti-cancer activity and hormons with anti-cancer activity.

It is preferred that these substances are administered during those treatment intervals, when the patient recovers from the side effects of having been treated with a compound selected from the group consisting of cytostatica, biologicals with anti-cancer activity and hormons with anti-cancer activity.

This task has been solved by the provision of a combination therapy consisting of the administration of hydroxyalkylated starch(es) for the treatment of cancer by reducing tumor growth rates and the administration of a compound selected from the group consisting of cytostatica, biologicals with anti-cancer activity and hormons with anti-cancer activity. These may be administered at the same day or in alternating fashion at different time intervals. The administration of hydroxyalkylated substances may take place before administration of the cytostatica or after. A simultaneous administration, by administering a single composition comprising both substances is not part of the invention. One aspect of the invention is a kit providing for both substances, any cytostatic composition, preferably the standard-of-care drug, in one vial and the HAS, preferably as injectable solution in a second vial, both presented in a kit. Such a vial may be any pharmaceutically acceptable containment for the substances, such as for example a galss bottle, or a plastic bottle, or a plastic bag. The HAS solution may be in form of a pharmaceutical composition comprising a hydroxyalkylated starch as therapeutically active ingredient, resulting in a reduction of tumor growth rates, is provided for the treatment of cancer.

It could be shown that a combination of the hydroxyalkylated straches substances and the compound selected from the group consisting of cytostatica, biologicals with anti-cancer activity and hormons with anti-cancer activity has a stronger growth rate limiting effect on tumors, than the standard of treatment with such a compound alone, whilst not harming normal cells.

While HAS solutions have been administered to a high number of humans (without tumors) without showing any severe side effect, it was now for the first time noticed that these substances might have an anti-proliferative effect selectively on tumor cells.

It has been shown that the combined administration of hydroxyalkylated starch and a compound selected from the group consisting of cytostatica, biologicals with anti-cancer activity and hormons with anti-cancer activity to a subject suffering from cancer in form of a growing tumor, inhibited its further progression by reducing the size of the tumor, associated with said cancer, compared to the size of tumors in subjects treated with this compound alone, measured at the end of the treatment phase.

This significant therapeutic effect of the combination of administered cytostatica and administered hydroxyalkyl starches in reducing tumor growth rate, whilst not causing a decreased general health status, indicated by a loss of body weight, has been demonstrated herein for the first time. To our knowledge, no one has ever reported upon an increased therapeutic effect on a cancer or a tumor by combining the administration, preferably i.v. administration of a compound selected from the group consisting of cytostatica, biologicals with anti-cancer activity and hormons with anti-cancer activity, with the administration, preferably the i.v. administration of hydroxyalkylated starch itself. Whilst Hydroxyalkylated starches have been proposed as stabilizing agents or solubilisers or osmotically active ingredients, it has never been shown that the application of HAS itself has an additional tumor growth rate reducing, or anti-proliferative effect when given in a combination therapy with a cytostatic.

According to the invention hydroxyalkylated starch(es) are provided as therapeutically active compounds for improving the treatment of cancer, characterized by the presence of growing tumors, which are therapeutically effective in reducing the tumor growth rate, more effectively than the compounds selected from the group consisting of cytostatica, biologicals with anti-cancer activity and hormons with anti-cancer activity, alone, preferably whilst not affecting a normally proliferating cell..

According to the current application an "alkyl group" is understood to comprise a linear or branched functional group or side-chain that consists of saturated hydrocarbons, preferably of a chain length of 2 to 12 carbon atoms. Said saturated hydrocarbon can be linear (general formula -$C_nH_{2n+1}$) wherein the carbon atoms are joined in a snake-like structure, such as propyl-, butyl-, pentyl-, hexyl-, heptyl-, octyl-, nonyl-, decanyl-, undecanyl- and dodecanyl-residues; or branched (general formula -CnH2n + 1, wherein n is above or equal 3) wherein the carbon backbone splits off in one or more directions, comprising for example isopropyl-, isobutyl-, tert.-butyl, 1-isopentyl-, 2-isopentyl, 3-isopentyl-, neopentyl-rests.

According to the invention, the term "cancer" refers to a proliferative disorder or disease caused or characterized by the proliferation of cells which have lost susceptibility to normal growth control. The term encompasses a disease which is associated with the growing of tumors and any other cell proliferative disorders. According to the invention, the term is meant to include all pathological conditions involving uncontrolled growth of cells, irrespective of stage or of invasiveness.

In one embodiment the cancer may be localized to a specific tissue or organ (e.g. in the breast, the prostate or the lung), and, thus, may not have spread beyond the tissue of origin. In another embodiment the cancer may be invasive, and, thus may have spread beyond the layer of tissue in which it originated into the normal surrounding tissues (frequently also referred to as locally advanced cancer). Invasive cancers may or may not be metastatic. In a preferred embodiment the cancer is metastatic. A cancer is metastatic, if it has spread from its original location to distant parts of the body.

Further the term cancer is understood to describe all types of cancer known in the art.

The term "tumor" is meant to describe an accumulation of cells that are growing in an uncontrolled manner, an abnormally grown or growing body tissue, or an accumulation of proliferating cells. Tumors can be cancerous (malignant) or noncancerous (benign). A cell proliferative disease usually results in the occurrence of a tumor.

A "pharmaceutical composition" according to the invention comprises a therapeutically effective amount of a HAS, as described herein, which can be further substituted, for example via the hydroxyl function attached at the alkyl rest, or instead of this hydroxyl function, and preferably of all those HAS and HES that are specifically and explicitly disclosed, including thio-HAS and thio-HES.

The pharmaceutical composition may comprise solid or liquid formulations of different concentrations. Different embodiments comprising the hydroxyl alkylated starch either on its own or as a pharmaceutical composition are described in more detail below: According to the invention the active ingredient, hydroxyalkyl starch may be administered on its own, simply dissolved in an electrolytic solution, or it may be used in combination with a pharmaceutical excipient. Generally, the hydroxyalkyl starch itself will be in a solid form which can be combined with a suitable pharmaceutical excipient that can be in either solid or liquid form. As excipients, carbohydrates, inorganic salts, antimicrobial agents, antioxidants, surfactants, buffers, acids, bases, and combinations thereof may be mentioned. A carbohydrate such as a sugar, a derivatized sugar such as an alditol, aldonic acid, an esterified sugar, and/or a sugar polymer may be present as an excipient. Specific carbohydrate excipients include, for example: monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol, sorbitol (glucitol), pyranosyl sorbitol, myoinositol, and the like. The excipient may also include an inorganic salt or buffer such as citric acid, sodium chloride, potassium chloride, sodium sulfate, potassium nitrate, sodium phosphate monobasic, sodium phosphate dibasic, and combinations thereof.

The pharmaceutical composition according to the present invention may also comprise an antimicrobial agent for preventing or determining microbial growth, such as, e.g., benzalkonium chloride, benzethonium chloride, benzyl alcohol, cetylpyridinium chloride, chlorobutanol, phenol, phenylethyl alcohol, phenylmercuric nitrate, thimersol, and combinations thereof.

The pharmaceutical composition according to the present invention may also comprise an antioxidant, such as, e.g., ascorbyl palmitate, butylated hydroxyanisole, butylated hydroxytoluene, hypophosphorous acid, monothioglycerol, propyl gallate, sodium bisulfite, sodium formaldehyde sulfoxylate, sodium metabisulfite, and combinations thereof.

The pharmaceutical composition according to the present invention may also comprise a surfactant, such as, e.g., polysorbates, or pluronics sorbitan esters; lipids, such as phospholipids and lecithin and other phosphatidylcholines, phosphatidylethanolamines, acids and fatty esters; steroids, such as cholesterol; and chelating agents, such as EDTA or zinc.

The pharmaceutical composition according to the present invention may also comprise acids or bases such as, e.g., hydrochloric acid, acetic acid, phosphoric acid, citric acid, malic acid, lactic acid, formic acid, trichloroacetic

acid, nitric acid, perchloric acid, phosphoric acid, sulfuric acid, fumaric acid, and combinations thereof, and/or sodium hydroxide, sodium acetate, ammonium hydroxide, potassium hydroxide, ammonium acetate, potassium acetate, sodium phosphate, potassium phosphate, sodium citrate, sodium formate, sodium sulfate, potassium sulfate, potassium fumarate, and combinations thereof. Generally, the excipient will be present in a pharmaceutical composition according to the present invention in an amount of 0.001 to 99.999 wt.-%, preferably from 0.01 to 99.99 wt.-%, more preferably from 0.1 to 99.9 wt.-%, in each case based on the total weight of the pharmaceutical composition.

In a preferred embodiment the pharmaceutical composition which according to the invention, is used in a combination therapy with pharmaceutical compositions containing compounds selected from the group consisting of cytostatica, biologicals with anti-cancer activity and hormons with anti-cancer activity comprise the hydroxyalkyl starch, as described herein, as the only pharmaceutically active ingredient, , wherein HAS is therapeutically active against cancer, by reducing the tumor growth rate.

The term "combination therapy" according to this invention is meant to describe all situations wherein the treatment of a cancer patient involves a) the administration of one or more compounds selected from the group consisting of cytostatica, biologicals with anti-cancer activity and hormons with anti-cancer activity and b) the administration of HAS according to the invention, as tumor growth reducing agent. Treatment regimens wherein HAS is administered to achieve other effects than the tumor growth reduction as described herein, for example to treat hypovolemia are not comprised in this term.

The terms "treating cancer" and "treatment of cancer", refer to therapeutic measures, wherein the object is to prevent or to slow down (lessen) an undesired physiological change or disorder, such as the growth, development or spread of a hyperproliferative condition, such as a cell proliferative disease or a neoplastic disease, the forming of a benign or malignant tumor, or metastases therefrom, or cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, and remission whether partial or total. Metastatic cancer cells usually arise from a cell type disclosed herein and the major difference from the types disclosed herein is that these cells are now present in a tissue from which the cancer cells did not originally develop. Consequently, if a cancer type is mentioned the term encompasses its metastatic form.

It is to be understood that a treatment can also mean prolonging survival as compared to expected survival if not receiving treatment. It is to be understood that a treatment can also be understood as prevention of cancer or prevention of tumor growth.

In a preferred embodiment the treatment is effective to reduce the growth rate of tumors arising from metastatic cancers.

It is particularly envisaged that the term "treatment of cancer" according to the invention comprises the administration of a therapeutically effective amount of the aforementioned hydroxyalkylated starches in combination with a compound selected from the group consisting of cytostatica, biologicals with anti-cancer activity and hormons with anti-cancer activity resulting in at least one of the effects from the group consisting of reducing the number of cancer cells; reducing the tumor size; inhibiting i.e., slowing to some extent and preferably stopping cancer cell infiltration into peripheral organs; inhibiting i.e., slowing to some extent and preferably stopping tumor metastasis; inhibiting, at least to some extent, tumor growth; and relieving to some extent one or more of the symptoms associated with cancer; and increasing the quality of life. Whether a particular amount of the aforementioned compounds exerts at least one or several of these effects i.e. is pharmaceutically effective, can be determined by standard measures. Particularly, it can be determined by assessing cancer therapy efficacy. Cancer therapy efficacy, e.g., can be assessed by determining the time to disease progression, the increase of quality of life and/or by determining the response rate. Thus, the required dosage will depend on the severity of the condition being treated, the patient's individual response, the method of administration used, the cancer type, the tumor and the like. The skilled person is able to establish a correct dosage based on his general knowledge. Generally, the dose of the hydroxyalkylated starch component may also be administered independently from the state of the disease as the product is considered as non-toxic and dose limits are considered to be based on the current clinical experience (with e.g. Voluven® 10% solution of HES 130/0.4: 30ml/kg/day and/or Volulyte® 6% solution of HES 130/0.4: 50ml/kg/day).

The term "administering" as used herein, preferably, refers to the introduction of the hydroxyalkyl starch and a compound selected from the group consisting of cytostatica, biologicals with anti-cancer activity and hormons with anti-cancer activity according to the invention, or the pharmaceutical compositions according to the invention, into subjects, such as, for example, cancer patients. The term comprises methods for administering both particular compounds of the combination by parenteral and enteral routes of administration. The parenteral routes of administration are selected from the group comprising intravascular, transmucosal, trans-/intradermal, intramuscular (i.m.), intravenous (i.v.), intradermal, subcutaneous (s.c.), intraperitoneal (i.p.), intraventricular, intracranial, vaginal, nasal, intratumoral, intraosseal, intrathecal and intrapulmonal. The enteral methods of administration are selected from the group comprising oral, nasal, sublingual and rectal, administration and administration by gavage (via feeding tube), such as a percutaneous endoscopic gastrostomy (PEG tube) or percutaneous jejunostomy feeding tube (PJG tube). It is to be understood that the route of

administration may depend on the cancer to be treated.

According to the invention the preferred route of administration is the parenteral administration. It is further preferred that such parenteral route is an infusion, preferably into a blood vessel. The most preferred route of administration is an intravenous route.

Preferably, the administration of a single dose (bolus) of a therapeutically effective amount of the aforementioned compounds is over a period of 5 min to 5 h.

Hydroxyethylated starches for the treatment and prophylaxis of hypovolemia are in use, also as i.v. infusions, since many years and show no toxic side effects. The dose recommendations known from such other medical uses specify an upper limit due to physical limits only. Solutions of "6% Hydroxyethyl Starch 130/0.4" in 0.9% sodium chloride can be administered repetitively over several days. Hence the patient can be provided with continued infusions of HES in combination with single doses or multiple doses of one or more compounds selected from the group consisting of cytostatica, biologicals with anti-cancer activity and hormons with anti-cancer activity to treat his cancer and inhibit the growth rate of the tumor.

It is a preferred embodiment according to the invention that the administration of the substance HAS or a pharmaceutical composition comprising HAS is repeated according to the requirements of the patient who either thereafter or before or simultaneously receives a standard therapy based on administration of one or more compounds selected from the group consisting of cytostatica, biologicals with anti-cancer activity and hormons with anti-cancer activity.

In another preferred embodiment the therapeutically effective substance according to the invention is administered continuously while the patient either thereafter or before or within the time of continuous administration receives a standard therapy based on administration of one or more compounds selected from the group consisting of cytostatica, biologicals with anti-cancer activity and hormons with anti-cancer activity.

The hydroxyl alkylated starches may also be used in the so called watering therapy during or following a cancer treatment regimen with e.g. cytostatics (Ko et al. (2011) Intravenous fluid therapy successfully prevents renal injury by gemcitabine in patients with pancreatic cancer. Pancreas 2011 Jul; 40(5): 784-6). Here an additional benefit of treating cancer, may be achieved besides the protection of organs e.g. kidneys and bladder, which is achieved by administering the watering solutions.

Preferably the hydroxyl alkylated starches are administered before the standard treatment regimen with one or more compounds selected from the group consisting of cytostatica, biologicals with anti-cancer activity and hormons with anti-cancer activity begins.

Preferably, the hydroxyalkyl starch is administered together with a suitable carrier, and/or a suitable diluent, such as preferably a sterile solution for i.v., i.m., i.p. or s.c. application. It is further preferred that the route of administration involves a ready-to-use liquid solution of the HAS.

It is also preferred that the HAS or HES according to the invention is contained in a pharmaceutically acceptable container and that it is combined in a kit together with a pharmaceutical composition of a compound selected from the group consisting of cytostatica, biologicals with anti-cancer activity and hormons with anti-cancer activity drug. It is also preferred that the HAS therein is provided as an aqueous solution. It is further preferred that the aqueous solution is provided in a pharmaceutically acceptable device. Such a device may for example be in form of a syringe or bottle or a bag. The person skilled in the art will be able to select a suitable material. For example, a bottle may be made of glass or plastic materials and a bag may be made from plastic materials suitable and/or authorized for the containment of drugs. Preferably such an aqueous solution will be provided in a pharmaceutically acceptable bag suitable for the containment of drugs and/or the i.v. administration to patients. It is especially preferred that such a solution is provided in a plastic bag, such as for example the "freeflex bag".

The term "therapeutically effective amount", as used herein, preferably refers to an amount of the hydroxyalkyl starch as defined herein, or the pharmaceutical composition according to the present invention that in combination with one or more compounds selected from the group consisting of cytostatica, biologicals with anti-cancer activity and hormons with anti-cancer activity(a) reduces or inhibits tumor growth rate, (b) treats the cancer, or (c) attenuates, ameliorates, or eliminates the cancer, or (d) prevents the spread of cancer (metastasis), or (e) prevents the generation of cancer or (f) reduces tumor burden. More preferably, the term refers to the amount of the pharmaceutical composition comprising hydroxyalkyl starch as the only active ingredient as defined herein, administered in a combination therapy according to the present invention that (a) reduces or inhibits tumor growth rate, (b) treats the cancer, or (c) attenuates, ameliorates, or eliminates the cancer. Preferably this combination therapy is more effective than the treatment with one or more compounds selected from the group consisting of cytostatica, biologicals with anti-cancer activity and hormons with anti-cancer activity alone. More preferably this treatment regimen of the combination of hydroxalkyl starches and one or more compounds selected from the group consisting of cytostatica, biologicals with anti-cancer activity and hormons with anti-cancer activity is less straining for the patient and causes less side effects, than a treatment regimen with one or more compounds selected from the group consisting of cytostatica, biologicals with anti-cancer activity and hormons with anti-cancer activity alone in a dosage required to achieve the same tumor growth reduction effect.

The term "subject", as used herein, relates to animals and, preferably, to mammals. In a preferred embodiment,

the subject is a rodent such as a mouse or a rat. Even more preferred is the embodiment, wherein the subject is a primate. Most preferably, the subject is a human. According to the invention it is understood that the term "subject" also relates to an individual suffering from cancer or an individual in need of cancer treatment. In a preferred embodiment of the invention the term "subject" describes a cancer patient.

According to the invention the term "carcinoma" describes cancer types that arise in epithelia tissue, in the skin or in tissues that line or cover internal organs. According to the invention the cancer is preferably selected from the group consisting of skin, lung, colon, pancreatic and epithelial, squamous and basal cell carcinomas, melanomas, papillomas, and adenomas. Most preferably the cancer is selected from the group consisting of breast carcinoma, prostate carcinoma, lung carcinoma and renal carcinoma.

According to the invention the term "sarcoma" describes cancer types that usually arise from bone, cartilage, fat, muscle, blood vessels, or other connective or supportive tissue. According to the invention the cancer is preferably selected from the group consisting of bone cancer, soft tissue cancers, osteosarcoma, synovialsarcoma, liposarcoma, angiosarcoma, rhabdosarcoma, chondrosarcoma and fibrosarcoma.

According to the invention the term cancer also comprises cancer types that arise in the tissues of the brain and spinal cord. Preferably the cancer is selected from the group consisting of brain and spinal cord tumors, gliomas, meningiomas, pituitary adenomas, vestibular schwannomas, primary CNS lymphomas, and primitive neuroectodermal tumors.

The term "hydroxyalkyl starch" or "hydroxy alkylated starch" encompasses various hydroxyl-alkylated starches, as will be described in more detail below. These hydroxyalkyl starches may be further substituted.

Hydroxyalkyl starch is an ether derivative of partially hydrolyzed natural starches, in which hydroxyl groups in the starch are suitably hydroxyalkylated. Preferred hydroxyalkyl starches are hydroxypropyl starch and hydroxyethyl starch, with hydroxyethyl starch being especially preferred.

The current invention not only comprises a new medical use of hydroxyalkylated starches (HAS) that are substituted with an alkyl residue which carries a hydroxy function, but also those alkylated starches that are substituted with alternative alkyl groups. In one embodiment the alkyl groups carry thiol groups, also referred to as sulfhydryl group. In another embodiment those alkylated starches have the unsubstituted hydroxy functions (hydroxyl groups) in the glucose unit replaced by thio functions (thiol groups). In another embodiment, some of the glucose units of the alkylated starches are alkylated, wherein some of these alkylgroups carry thiol groups, and some carry hydroxyl functions, and wherein some of the C2, C3 and C6 positions may be substituted, preferably by thiol groups. These starches are referred to herein as thio-HAS. They have been described in more detail below and in PCT/EP2011/003458.

Starch is a polysaccharide of the formula $(C_6H_{10}O_5)_n$ which is composed substantially of alpha-D-glucose units, coupled via glycoside linkages. Generally speaking, starch consists substantially of amylose and amylopectin. Amylose is composed of linear chains in which the glucose units are linked via alpha-1,4-glycosidic linkages. Amylopectin has a highly branched structure, with alpha-1,4-glycosidic linkages and alpha-1,6-glycosidic linkages.

Natural starches from which hydroxyalkyl starches may be prepared include cereal starches, grain legume starches and potato starches. Cereal starches include rice starches, wheat starches such as einkorn starches, spelt starches, soft wheat starches, emmer starches, durum wheat starches or kamut starches, maize starches, rye starches, oat starches, barley starches, triticale starches and millet starches such as sorghum starches or teff starches. Grain legume starches include bean starches, pea starches, lentil starches and lupine starches. Preferred natural starches from which hydroxyalkyl starches are prepared have a high content of amylopectin relative to amylose. The amylopectin content of these starches is, for example, at least 70% by weight, preferably at least 75% by weight, more preferably at least 80% by weight, more preferably at least 85% by weight, more preferably at least 90% by weight, such as up to 95% by weight, up to 96% by weight, up to 97% by weight, up to 98% by weight or up to 99% by weight or up to 100% by weight. Natural starches having an especially high amylopectin content are, for example, suitable potato starches such as waxy potato starches, which are preferably extracted from substantially amylose-free potatoes, which are either traditionally cultivated, e.g. the natural variety Eliane, or genetically modified amylopectin potato varieties, and starches from waxy varieties of cereals such as waxy maize or waxy rice.

Generally, hydroxyalkyl starch is prepared by breaking starch grains and cleaving the macromolecules to obtain molecules having a desired size. Cleaving can be carried out, for example, by enzymatic degradation, as for example using alpha-amylase and/or beta-amylase, and/or by means of acidic hydrolysis. Purification of the desired fractions can be accomplished, for example, by means of ultrafiltration, using membranes having a suitable cut-off limit, which allow the separation, for example, of low-molecular by-products having a molecular weight of up to 5000 Da or up to 1000 Da. Two or more cleaving stages can be carried out in series, with the possibility in each stage of using the same or different cleaving technologies. After each cleaving stage, the product obtained can be purified. The product ultimately obtained can be isolated, as for example by freeze-drying.

On the basis of the starch fractions thus obtained, hydroxyalkyl starch is prepared by etherification of hydroxyl groups. In general, all reactions known from the etherification of low-molecular alcohols may be contemplated, such as reactions without catalyst or with basic catalysts. The preferred methods in technical processes include the Michael

addition of activated olefins, the Williams synthesis with nucleophilic substitution of compounds containing aliphatic halogen, or the reaction with oxiranes, also known as epoxides.

Concerning the preparation of hydroxyalkyl starch, more particularly of hydroxyethyl starch, reference is made, for example, to Sommermeyer et al., Chromatographia, 25, 1988, pp. 167-168; C. Jungheinrich et al., Clin. Pharmacokin., 44 (7), 2005, pp. 681-699; J.-M. Mishler IV, Pharmacology of hydroxyethyl starches, Oxford Medical Publications, 2002, pp. 1-30.

According to the present invention, the term "hydroxyalkyl starch" (HAS) refers to a starch derivative having a constitution according to the following formula (III)

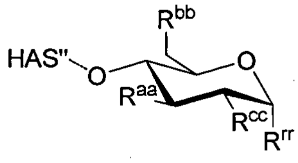

(III)

wherein the depicted ring structure is either a terminal or a non-terminal saccharide unit, which may be one anhydroglucose unit as described separately in this application, of the HAS molecule and wherein HAS" is a remainder, i.e. a residual portion of the hydroxyalkyl starch molecule, said residual portion forming, together with the depicted ring structure containing the residues $R^{aa}$, $R^{bb}$ and $R^{cc}$ and $R^{rr}$ the overall HAS molecule. In formula (III), $R^{aa}$, $R^{bb}$ and $R^{cc}$ are independently of each other hydroxyl, a linear or branched hydroxyalkyl group or-O-HAS".

Residue $R^{rr}$ is -O-HAS" in case the depicted ring structure is a non-terminal saccharide unit of the HAS molecule. In case the depicted ring structure is a terminal saccharide unit of the HAS molecule, $R^{rr}$ is -OH, and formula (III) shows this terminal saccharide unit in its hemiacetal form. This hemiacetal form, depending on e.g. the solvent, may be in equilibrium with the free aldehyde form as shown in the scheme below:

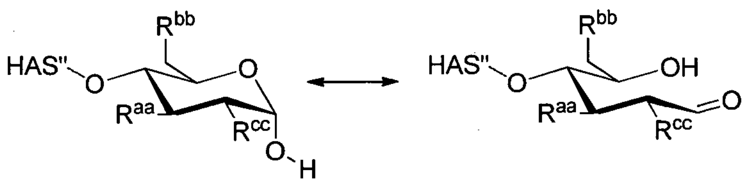

The term O-HAS" as used in the context of the residue R" as described above is, in addition to the remainder HAS" shown at the left hand side of formula (III), a further remainder of the HAS molecule which is linked as residue $R^{rr}$ to the depicted ring structure of formula (III)

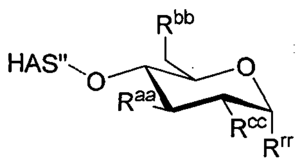

(III)

and forms, together with the residue HAS" shown at the left hand side of formula (III) and the depicted ring structure the overall HAS molecule.

Each remainder HAS" discussed above comprises, preferably essentially consists of - apart from terminal saccharide units - one or more repeating units according to formula (IIIa)

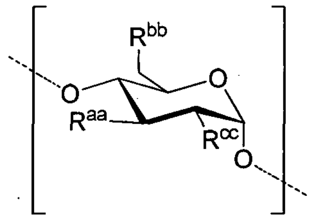

(IIIa)

According to the present invention, the HAS molecule shown in formula (III) is either linear or comprises at least one branching point, depending on whether at least one of the residues $R^{aa}$, $R^{bb}$ and $R^{cc}$ of a given saccharide unit comprises yet a further remainder -O-HAS". If none of the $R^{aa}$, $R^{bb}$ and $R^{cc}$ of a given saccharide unit comprises yet a further remainder -O-HAS", apart from the HAS" shown on the left hand side of formula (III), and optionally apart from HAS" contained in $R^{rr}$, the HAS molecule is linear.

Hydroxyalkyl starch comprising two or more different hydroxyalkyl groups is also conceivable. The at least one hydroxyalkyl group comprised in the hydroxyalkyl starch may contain one or more, in particular two or more, hydroxyl groups. According to a preferred embodiment, the at least one hydroxyalkyl group contains only one hydroxyl group.

According to the present invention, a hydroxyalkyl starch (HAS) according to the above-mentioned formula (III)

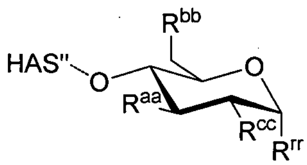

(III)

is disclosed for the treatment of cancer. The saccharide units comprised in HAS", apart from terminal saccharide units, may be the same or different, and preferably have the structure according to the formula (IIIa)

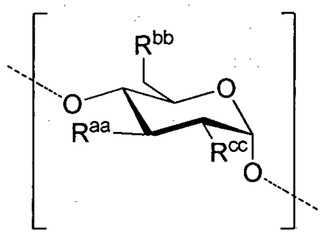

(IIIa)

as shown above. This unit is also described in more detail in the following:

A typical anhydroglucose unit of a hydroxyalkyl starch molecule has the following formula (I):

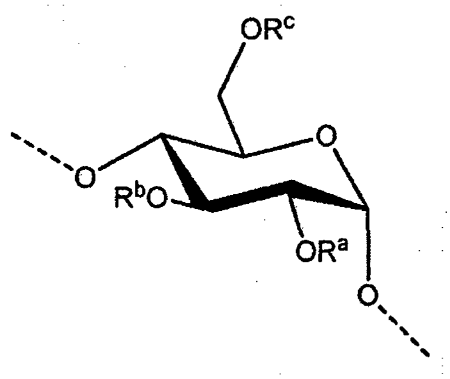

(I)

In formula (I), the residues $R^a$(-$OR^a$ is depicted as $R^{cc}$ in formula III), $R^b$(-$OR^b$ is depicted as $R^{aa}$ in formula III) and $R^c$(-$OR^c$ is depicted as $R^{bb}$ in formula III) are independently $[(-CR^jR^k)_y-O]_z-H$, in which $R^j$ and $R^k$ are independently H or alkyl, preferably lower alkyl such as methyl or ethyl, preferably H;
y is an integer from 0 to 6, preferably from 2 to 4 such as 0, 1, 2, 3, 4, more preferably 2 or 3, more preferably 2;
z is an integer from 0 to 20, preferably from 0 to 10, more preferably from 0 to 6, more preferably from 0 to 4 such as 0, 1, 2, 3, 4, with the proviso that in case y is 0, z is likewise 0.

If there is a branching site of the macromolecule located on the glucose molecule, however, $R^c$ may also be a further chain of glucose molecules, such as, for example, $(Glc-1,4-Glc)_n-Glc$, in which n may have a value from 0 to 20. The anhydroglucose units in such a side-chain may also be substituted, like the chain identified initially.

If the anhydroglucose unit is a unit of the hydroxyalkyl starch molecule which is not substituted by at least one hydroxyalkyl moiety, then the index z in $R^a$ and $R^b$ and $R^c$ is 0. If the anhydroglucose unit is a unit of the hydroxyalkyl starch molecule which is substituted by a hydroxyalkyl moiety in C2 position only, the index z is 0 in $R^b$ and $R^c$ and is greater than 0 in $R^a$. If the anhydroglucose unit is a unit of the hydroxyalkyl starch molecule which is substituted by a hydroxyalkyl moiety in C3 position only, the index z is 0 in $R^a$ and R° and is greater than 0 in $R^b$. If the anhydroglucose unit is a unit of the hydroxyalkyl starch molecule which is substituted by a hydroxyalkyl moiety in C6 position only, the index z is 0 in $R^a$ and $R^b$ and greater than 0 in $R^c$. If the anhydroglucose unit is a unit of the hydroxyalkyl starch molecule which is substituted by a hydroxyalkyl moiety in C2 and C3 position only, the index z is 0 in $R^c$ and is greater than 0 in $R^a$ and $R^b$. If the anhydroglucose unit is a unit of the hydroxyalkyl starch molecule which is substituted by a hydroxyalkyl moiety in C2 and C6 position only, the index z is 0 in $R^b$ and greater than 0 in $R^a$ and $R^c$. If the anhydroglucose unit is a unit of the hydroxyalkyl starch molecule which is substituted by a hydroxyalkyl moiety in C3 and C6 position only, the index z is 0 in $R^a$ and greater than 0 in $R^b$ and $R^c$. If the anhydroglucose unit is a unit of the hydroxyalkyl starch molecule which is substituted by a hydroxyalkyl moiety in C2 and C3 and C6 position, the index z is greater than 0 in $R^a$ and $R^b$ and $R^c$.

In one embodiment according to the invention the hydroxyalkyl starch is a pure hydroxypropyl starch, herein a respective residue $R^a$ or $R^b$ or $R^c$ with an index z greater than 0 has an index y of 3, and both $R^j$ and $R^k$ are H. Since multiple hydroxypropylation may occur during the preparation, the index z can be greater than 1, such as 2, 3 or more.

In addition, whenever the alkylation is carried out using epoxides a further form of the side-chain is formed. In this case, the hydroxy function is not located on the terminal C atom of the alkyl side-chain, but instead on $C^2$, i.e. the second C atom, countng from the ring. Following a propylation by means of the epoxide 1,2-epoxypropane, at least one of the residues $R^a$ or $R^b$ or R° would have the following appearance, for example: $(C^1R^jR^k-C^2R_j(OH)-C^3R^jR^kH)$. After propylation by means of an unsubstituted 1,2-epoxypropane, in other words with methyloxirane ("propylene oxide"), $R^j$ and $R^k$ each are H.

In a preferred embodiment, the hydroxyalkyl starch is a pure hydroxyethyl starch, here a respective residue $R^a$ or $R^b$ or $R^c$ with index z greater than 0 has an index y which is 2, and both $R^j$ and $R^k$ are H. Since multiple hydroxyethylation may occur during the preparation, the index z can be greater than 1, such as 2, 3 or more. If, for example, a double hydroxyethylation takes place on a given hydroxyl group of an anhydroglucose unit, the index y and the index z are both 2, and the residues $R^j$ and $R^k$ are both H in one respective residue $R^a$ (or $R^b$ or $R^c$), which is, accordingly, $-CH_2-CH_2-O-CH_2-CH_2-OH$.

It is also possible that different alkylating agents are used (mixed alkylation), which means that $R^a$, $R^b$ and $R^c$ are alternatively to be represented in such a way that y may have different values - accordingly, for example, in the case of mixed hydroxyethylation and hydroxypropylation, y may be 2 in one residue and 3 in the other residue. Furthermore, in a residue R with z > 0, there may be a side-chain in which the value y may have different values, e.g. 2 or 3.

Mixed alkylation with epoxides may also result in the possible presence of structural units of the form $[(-CR^jR^k)_y-O]_z-H$ and of the form $[-C^1R^jR^k-C^2R^j(C^3R^jR^kH)-O]_z-H$ in one or various residues $R^a$, $R^b$ or $R^c$ in different numbers.

Furthermore, the glucose polymer may also be substituted by a thioalkyl residue. In principle, therefore, it is also possible for the above-described embodiments to exist with a sulphur atom instead of an oxygen atom in the substituted side chain. In this case, at least one of the residues $R^a$, $R^b$ and $R^c$ may be $-[(-CR^jR^k)_y-S]_z-H$ or $[-C^1R^jR^k-C^2R^j(C^3R^jR^kH)-S]_z-H$. According to the invention, thiohydroxyalkyl starches of this kind are likewise disclosed for the treatment of cancer.

Processes for preparing thiohydroxyalkyl starches can be found in the PCT Application "Conjugates comprising Hydroxyalkyl Starch and a Cytotoxic Agent and Process for their Preparation" published in January 2012, WO2012/004005 (PCT/EP2011/003458); in particular, reference is made to the preparation processes on pages 245-252 (beginning with "1.3. Special Procedures" up to and including "1.4.9. General procedure for the synthesis of SH-HES using sodium sulfide as nucleophile") and, where necessary for comprehension, to the associated tables 6 to 9 on pages 259-263.

In a preferred embodiment the hydroxyalkyl starch according to the invention is hydroxyethyl starch, hydroxypropyl starch or hydroxybutyl starch, with hydroxyethyl starch being particularly preferred.

According to the present invention, the hydroxyalkyl starch (HAS) is preferably a hydroxyethyl starch (HES), the hydroxyethyl starch preferably having a structure according to the following formula (III)

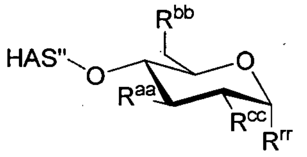

(III)

wherein $R^{aa}$, $R^{bb}$ and $R^{cc}$ are independently of each other selected from the group consisting of -O-HES", and $-[O-CH_2-CH_2]_s$,-OH, wherein s is in the range of from 0 to 4 and wherein HAS", is the remainder of the hydroxyethyl starch and is abbreviated with HES". Residue $R^{rr}$ is either -O-HES" or, in case the formula (III) shows the terminal saccharide unit of HES, $R^{rr}$ is -OH.

As a polymer, and owing to the preparation processes, hydroxyalkyl starch is a polydisperse compound in which the individual hydroxyalkyl starch molecules may differ with respect to the degree of polymerization, the number and the pattern of the branching sites, and the substitution pattern, i.e. the number and/or sites of the hydroxyalkyl groups. Therefore, hydroxyalkyl starch is usually characterized by statistically averaged parameters. These are, generally, the average molecular weight and parameters which characterize the substitution pattern. The latter parameters are typically identified as degree of substitution (DS), molecular substitution (MS) and C2/C6 ratio, i.e. the ratio of the number of anhydroglucose units substituted in C2 position to the number of anhydroglucose units substituted in C6 position, or the ratio of Mw relative to Mn (Mw/Mn), which is usually referred to as PDI (polydispersity index) and characterizes the spread of the molecular weight distribution.

Hydroxyalkyl starch may be substituted with hydroxyalkyl groups not only at the C2 and C6 sites, but also at the C3 site, but this information is usually omitted when referring to a specific type of HAS.

The second parameter specifying a HAS usually refers to the degree of molecular substitution MS and the third parameter either refers to the ratio of substitutions at C2 versus substitutions at C6 (C2/C6 ratio) or to the PDI.

Generally speaking, there are two ways of statistically describing the average molecular weight of hydroxyalkyl starch. The first parameter is the number-average molecular weight, commonly referred to as Mn or $M_n$; the second parameter is the weight-average molecular weight, commonly referred to as Mw or $M_w$.

The molecular weight can be determined, for example, by means of gel permeation chromatography with multiple-angle light-scattering detection (GPC/MALLS/RI). Reference is made, for example, to W.-M. Kulicke et al., Starch, 45 (12), 1993, pp. 445-450. Alternatively, the molecular weight can be determined using flow-FFF/MALLS, as for example in accordance with European Pharmacopoeia 7.0, 01/2011:1785, p. 984 or else by B. Wittgren et al., Int. J. Polym. Anal. Charact. 7 (1-2), 2002, pp. 19-40.

In this context the number average molecular weight is defined by equation 1:

$$\overline{M}_n = \frac{\sum_i n_i \cdot M_i}{\sum_i n_i}$$

(1)

in which $n_i$ is the number of hydroxyalkyl starch molecules of species i having molar mass $M_i$. $\overline{M}_n$ indicates that this is an average value, but the line is typically omitted.

The weight average molecular weight $M_w$ is defined by the following equation:

$$\overline{M}_w = \frac{\sum_i n_i \cdot M_i^2}{\sum_i n_i M_i}$$

(2)

in which $n_i$ is the number of hydroxyalkyl starch molecules of species i having molar mass $M_i$. $\overline{M}_w$ indicates that this is an average value, but the line is typically omitted.

In the context of the present description the term "mean molecular weight" refers to the weight determined by the MALLS (multiple angle laser light scattering)-GPC method.

Hydroxyalkyl starches according to the invention have a mean molecular weight (Mw or MW) varying from as low as about 20 kDa to mean molecular weights up to 1300 kDA.

The ratio of Mw relative to the Mn (Mw/Mn), which is usually referred to as PDI, polydispersity index, is a parameter characterizing the spread of the molecular weight distribution. The closer this parameter is to the value 1, the less dispers the molecular weight distribution is.

According to the invention typical PDI values are in the range of from 4.0 to 1.1.

The substitution pattern can be determined quantitatively, at least partially, using $^1$H NMR or by a more elaborate method, by means of high-resolution $^{13}$C NMR. Reference is made to Y. M. Liu et al., Chin. Chem. Lett. 13 (11), 2002, pp. 1097-1099, and to W.-M. Kulicke et al., Starch, 45 (12), 1993, pp. 445-450. In general there are three customary parameters which describe the degree of substitution of hydroxyalkyl starch.

The first parameter, which is identified as "DS" (degree of substitution), describes the ratio of the number of substituted anhydroglucose units to the total number of all the anhydroglucose units. In view of this definition, the theoretical maximum value of DS is 1.0.

The parameter DS can be determined, for example, in accordance with W. Banks et al., Br. J. Pharmac., 47, 1973, pp. 172-178, O. Larm et al., Starch, 33 (7), 1981, pp. 240-244, or Sommermeyer et al., Starch, 44 (6), 1992, pp. 215-218.

The second parameter, which is typically identified as "MS" (molecular substitution), describes the ratio of the number of hydroxyalkyl residues (in mol) which have been added by hydroxyalkylation to the glucose molecules of the starch macromolecule, relative to the number of glucose monomers in the molecule.

Assuming that the alkylation results in the addition of a single alkyl unit per hydroxy function, the degree of molar substitution indicates what proportion of the three hydroxy units the glucose units on the starch molecule have been substituted or replaced by hydroxyalkyl units. Herein a substitution degree of 1 equals a 100% of substitution of one of the three free hydroxy groups. Hence theoretically the range of substitution could vary from 0.1 to 3, wherein three indicated that all three hydroxy units would be 100% substituted. There is a number of different types of HAS in the market, and their substitution degrees vary from 0.3 to 2.

The parameter MS may be determined in accordance with Ying-Che Lee et al., Anal. Chem. 55, 1983, pp. 334-338; or K. L. Hodges et al., Anal. Chem. 51, 1979, p. 2171. According to these methods, a known amount of the hydroxyalkyl starch is subjected to an ether cleavage in xylene, with addition of adipic acid and hydriodic acid. The

amount of iodoalkane released is subsequently determined by means of gas chromatography, using toluene as an internal standard and iodoalkane calibration solutions as external standards.

The third parameter, which is identified as the C2/C6 ratio, describes the ratio of the number of anhydroglucose units substituted in C2 position to the number of anhydroglucose units substituted in C6 position. During the preparation of the hydroxyalkyl starch, the C2/C6 ratio can be influenced via the amount of base used for the hydroxyalkylation reaction. Generally speaking, the higher the concentration of base, the greater the number of hydroxyl groups which are hydroxyalkylated in C6 position.

The parameter C2/C6 can be determined, for example, in accordance with Sommermeyer et al., Krankenhauspharmazie 8 (8), 1987, pp. 271-278, especially page 273.

Various types of hydroxyalkyl- and hydroxyethyl starch, therefore, are usually described by a statement of their average molecular weight, expressed in kDa, their degree of molar substitution (MS), and their degree of branching (C2/C6), or by an indication of their polydispersity (Mw/Mn).

The present invention provides a fundamentally new active therapeutic agent for the treatment of cancer, which reduces the problematic side effects associated with the administration of cancer therapeutics, especially of one or more compounds selected from the group consisting of cytostatica, biologicals with anti-cancer activity and hormons with anti-cancer activity, alone. In particular, the toxic side effects associated with the administration of one or more compounds selected from the group consisting of cytostatica, biologicals with anti-cancer activity and hormons with anti-cancer activity, indicated by a loss of body weight, as a response to the drastic treatment with cell toxic agents, can be reduced when treating the cancer with a combination therapy and thereby reducing the tumor growth rates with hydroxyalkylated starch (HAS) whilst also treating the patient with one or more compounds selected from the group consisting of cytostatica, biologicals with anti-cancer activity and hormons with anti-cancer activity, but preferably with a reduced dosing thereof.

The present invention therefore relates to a combination therapy wherein HAS is administered for the treatment of cancer as a first medicament, either administered before or after the administration of second medicament, which is characterized as being a therapeutically active compound selected from the group consisting of cytostatica, biologicals with anti-cancer activity and hormons with anti-cancer.In a further embodiment, the invention relates to a kit comprising a pharmaceutical composition comprising HAS according to the invention for the treatment of cancer and a second pharmaceutical composition comprising one or more therapeutically active compounds selected from the group consisting of cytostatica, biologicals with anti-cancer activity and hormons with anti-cancer activity. In a further embodiment the invention relates to methods of treatment of cancer comprising the administration of HAS according to the invention.

It was unexpectedly found that the combined administration of HAS and a therapeutically active compound selected from the group consisting of cytostatica, biologicals with anti-cancer activity and hormons with anti-cancer activity to mammals that were implanted with tumor tissue, and were therefore suffering from cancer, alleviated their symptoms and resulted in a more significant growth reduction of the tumor, whilst not adversely affecting their health or body weight when given in addition to one or more compounds selected from the group consisting of cytostatica, biologicals with anti-cancer activity and hormons with anti-cancer activity, than when these were administered alone.

Without wanting to be bound to any theory, it may be that the hydroxyalkylated starches affect the angiogenesis of the tumor and inhibit its further growth and the progression of cancer. Angiogenesis plays a critical role in the growth and spread of cancer. Tumors are depending from a growing network of capillaries, providing oxygen and nutrients. To grow a tumor requires the new formation of blood vessels. Without the ability to generate new blood vessels for provision of nutrients the non-angiogenetic neoplasia remain of a clinically irrelevant size and do not cause symptoms, the cancer cells cannot invade nearby tissue, to move throughout the body, and to form new colonies of cancer cells, called metastases. By inhibiting angiogenesis tumor dormancy is achieved. Hence anti-angiogenetic therapies try to block or reduce the blood circulation of the tumor by blocking or reducing its blood vessel building. It is possible that the polymeric substance of HAS blocks the newly formed blood vessels and thereby hinders the tumor to grow and the cancer to progress.

Advantageously, the hydroxyalkyl starch or the pharmaceutical composition comprising said hydroxyalkyl starch is not toxic and triggers hardly any side effects when given intravenously, which is of great advantage when compared to a cytotoxic agent. Hence wherein the max. dosage of a conventional cytotoxic agent is limited severely by its toxic side effects, a patient can receive repeated doses or continuous infusion of hydroxyethyl starches on a daily basis in addition to this treatment with a one or more compounds selected from the group consisting of cytostatica, biologicals with anti-cancer activity and hormons with anti-cancer activity. Ideally, the dosage of these one or more compounds selected from the group consisting of cytostatica, biologicals with anti-cancer activity and hormons with anti-cancer activity, can be reduced, due to the therapeutic effect of the administered HAS.

In one preferred embodiment of the invention the subject treated or in need of treatment already receives a cytotoxic agent or similar standard therapy, and receives hydroxyalkyl starch according to the invention as secondary treatment. This may either be administered in parallel to the standard treatment or alternating with the standard treatment, which may comprise chemotherapy or radiation therapy. It may be advised to treat the subject in need thereof with HAS for the inhibition of cancer whenever the subject requires a break from chemotherapeutics due to the severe side effects

caused by the standard treatment.

It is preferred that the hydroxyalkyl starch that is used ina combination therapy with one or more compounds selected from the group consisting of cytostatica, biologicals with anti-cancer activity and hormons with anti-cancer activity has a mean molecular weight MW of above 20 kDa, preferably above 40 kDa, and even more preferably a MW greater than 65 kDa. Preferably the MW is also not higher than 1300 kDa. More preferably the MW is in the range of from 75 to 1200 kDa, and more preferably in the range of from 90 to 800 kDa.

In one embodiment, the hydroxyalkyl starch (HAS) according to the invention has a molar substitution degree MS of the HAS in the range of from 0.1 to 1.5. Preferred embodiments comprise particular ranges of molar substitutions values of 0.15 to 1.5, 0.2 to 1.5, 0.3 to 1.5, 0.4 to 1.5, 0.5 to 1.5, 0.6 to 1.5, 0.7 to 1.5, 0.75 to 1.5, more preferably in the range of 0.1 to 1.3, 0.1 to 1.0, 0.1 to 0.8, 0.1 to 0.6, and 0.1 to 0.5 and also preferably in the range of from 0.90 to 1.4, such as 0.90, 0.95, 1.0, 1.05, 1.1, 1.15, 1.2, 1.25, 1.3, 1.35 or 1.4. A particularly preferred range is from 0.1 to 1.0, more preferably from 0.1 to 0.6, more preferably from 0.25 to 0.55.

According to an especially preferred embodiment, the hydroxyalkyl starch derivative has a mean molecular weight MW in the range of from 80 to 1200 kDa and a MS in the range of from 0.1 to 1.5. Preferred embodiments comprise particular ranges of molar substitutions values of 0.15 to 1.45, 0.3 to 1.45, 0.45 to 1.45, 0.6 to 1.45, 0.7 to 1.45, 0.75 to 1.45, more preferably in the range of 0.1 to 0.5 and preferably in the range of from 0.90 to 1.4, such as 0.90, 0.95, 1.0, 1.05, 1.1, 1.15, 1.2, 1.25, 1.3, 1.35 or 1.4, more preferably a molar substitution MS in the range of from 0.1 to 1.30, or 0.1 to 0.5.

In an especially preferred embodiment, the hydroxyalkyl starch derivative has a mean molecular weight MW in the range of from 30 to 700 kDa and a molar substitution in the range of from 0.1 to 0.7; more preferably a mean molecular weight MW in the range of from 80 to 700 kDa and a MS in the range of from 0.1 to 0.7.

In one embodiment the C2/C6 ratio of HAS, is in the range of from 0.5 to 20, more preferably in the range of from 2 to 20, 18, 2 to 17, 2 to 14, 2 to 12, 2 to 10, 2 to 8, 2 to 6, 2 to 5, or 2 to 4,. In another preferred embodiment said C2/C6 substitution is in the range of from 4 to 12, 6 to 12, 7 to 12, or preferably in the range of from 7 to 10, more preferably in the range from 8 to 9. In another preferred embodiment said C2/C6 substitution is in the range of from 4 to 6, more preferably is 5.7.

In a preferred embodiment, the polydispersion index PDI is in the range of from 1.1 to 4.0, more preferably in the range of from 1.1 to 3.5, 1.1 to 3, 1.1 to 2.5, 1.1 to 2, 1.1 to 1.5, 1.1 to 1.4, 1.1 to 1.3 and 1.1 to 1.2. In another preferred embodiment the PDI is in the range of from 1.2 to 4, 1.35 to 4, 1.5 to 4, 1.7 to 4, 1.8 to 4, 1.9 to 4, 2 to 4, 2.5 to 4 or 2 to 4, or 1.4 to 3.0.

All of these ranges are considered to comprise values that differ from the precise numbers given by about a tenth of their numeric value.

Preferably, the hydroxyalkyl starch according to the invention, in particular the hydroxyethyl starch, as described above, has a mean molecular weight MW (weight mean) above the renal threshold.

In another preferred embodiment the hydroxyalkyl starch according to the invention, in particular the hydroxyethyl starch, as described above, has a mean molecular weight MW (weight mean) below the renal threshold.

The renal threshold is determined according to the method described by Waitzinger et al. (Clin. Drug Invest. 1998; 16: 151-160) and reviewed by Jungheinrich et al. (Clin. Pharmacokinet. 2006; 44(7): 681-699). Preferably, the renal threshold is denoted to mean a molecular weight MW equal to or higher than 40 kDa, or 45 kDa or 60 kDa or 65kDa.

In the following, hydroxyalkyl starch structures are described in more detail, which comprise several different preferred embodiments of the described class of HAS, which is used in a combination therapy with one or more compounds selected from the group consisting of cytostatica, biologicals with anti-cancer activity and hormons with anti-cancer activity.

In one preferred embodiment the hydroxyalkylated starch is a hydroxyethylated starch known under the name "HES 130/0.4". Despite the name "HES 130/0.4" this is a hydroxyethylstarch with a mean molecular weight of 105 kDa, according to the standard measurement and calibration method described in European Pharmacopoeia 7.0, 01/2011:1785, p.984, with a molar substitution degree of 0.38--0.45, a mean molar substitution degree of 0.42. Its C2/C6 ratio is between 8.0 and 12.0. Its PDI is about 2, i.e. between 1.7 and 2.3. It is commercially available, for example as a 10% solution in 0.9% NaCl solution, under the registered trade name Voluven®. The difference between the value of the MW 130 of the publicly known specification of "HES 130/0.4" and the amended specification to HES 105/0.4 results from a change in the method calibration used for determining the Mw of HAS. Whereas previously the determination was performed according to Sommermeyer et al. (Krankenhauspharmazie, 8, 1987, 08, p. 271-278), the amended value (Mw 105) has been determined according to the calibration as described in European Pharmacopoeia 7.0, 01/2011:1785, p.984. The difference in the method is the value of the light scattering value dn/dc, wherein in the Sommermeyer method a dn/dc value of 0.135 is used, this value changed to 0.147+/-0.001 in the "Pharmacopoeia method".

Another preferred embodiment according to the invention is a hydroxyethylated starch known as "HES 100/1.0/1.3". This is a hydroxyethylstarch with a mean molecular weight of 100 kDa, determined according to Sommermeyer et al.; and with a mean molecular weight of about 84 kDa (75-93 kDa), determined according to European

Pharmacopoeia 7.0, 01/2011:1785, p.984; and a molar substitution degree of 1.0±0.05. Its C2/C6 ratio is 5.0 - 6.0 or preferably 5.7 and the PDI is 1.3±0.1.

Often the name indicated in parentheses such as "HES 200/0.5" refers to the old measurement, but it is explained herein, which Mw values will be generated if measured according to European Pharmacopeia (as cited before). The Mw values in the application (which are not part of the name) refer to those determined according to European Pharmacopoeia 7.0, 01/2011:1785, p.984 with the calibration method defined therein using a dn/dc value of 0.147-0.149, unless specifically mentioned otherwise.

It is another especially preferred embodiment according to the invention wherein said "HES 100/1.0/1.3" is further specified by one or more of the properties listed in Table 1 specifying the batch of "HES 100/1.0/1.3".

Table 1:

| Test parameter | Measured properties of "HES 100/1.0/1.3" used in Example 2 |
|---|---|
| Appearance | Solid |
| Colour | yellowish |
| Absorption 400 nm/lcm | 0.007 |
| Mw | 84 kDa +/- 8.4 kDa |
| Mw of the 10% smallest fraction | 31.700 Da |
| Mw of the 10% largest fraction | 177.567 Da |
| Mn | 64.883 Da |
| Mw/Mn (PDI) | 1.29 |
| MS | 0.99 |
| C2/C6 | 5.7 |

Another batch of a so called "HES 100/1.0/1.3" has been characterized as shown in this table 2:

Table 2:

| Test parameter | Measured properties of "HES 100/1.0/1.3" used in Example 5 |
|---|---|
| Mw | 78,4 kDa |
| Appearance | Solid |
| Colour | yellowish |
| Absorption 400 nm/1cm | 0.007 |
| Mw of the 10% smallest fraction | 24.977 Da |
| Mw of the 10% largest fraction | 178.700 Da |
| Mn | 55.993 Da |
| Mw/Mn (PDI) | 1.40 |
| MS | 1.02 |
| C2/C6 | 5.6 |

Another embodiment is a hydroxyethylated starch known as "HES 70/0.4/1.8" for treatment of cancer when used in the described combination therapy. This is a hydroxyethylstarch with a mean molecular weight of 70 kDa, a molar substitution degree of 0.4 and a PDI of 1.8.

Another embodiment is a hydroxyethylated starch known as "HES 70/0.5" for treatment of cancer when used in the described combination therapy. This is a hydroxyethylstarch with a mean molecular weight of 70 kDa, a molar substitution degree of 0.5.

Another embodiment is a hydroxyethylated starch HES 100/0.1/2.0 for treatment of cancer when used in the described combination therapy. This is a hydroxyethylstarch with a mean molecular weight of 100 kDa, a molar substitution degree of 0.1 and a PDI of 2.0.

Another embodiment is a hydroxyethylated starch named as "HES 100/0.1/2.0" for treatment of cancer when used in the described combination therapy. This is a hydroxyethylstarch with a mean molecular weight of 130 kDa, a molar substitution degree of 0.1 and a PDI of 2.0.

Another embodiment is a hydroxyethylated starch known as "HES 100/0.7/1.3" for treatment of cancer when used in the described combination therapy. This is a hydroxyethylstarch with a mean molecular weight of 100 kDa, a molar substitution degree of 0.7 and a PDI of 1.3.

Another embodiment is a hydroxyethylated starch known as "HES 100/1.0/1.1" for treatment of cancer when used in the described combination therapy. This is a hydroxyethylstarch with a mean molecular weight of 100 kDa, a molar substitution degree of 1.0 and a PDI of 1.1.

Another embodiment is a hydroxyethylated starch known as "HES 150/0.7/1.3" for treatment of cancer when used in the described combination therapy. This is a hydroxyethylstarch with a mean molecular weight of 150 kDa, a molar substitution degree of 0.7 and a PDI of 1.3.

Another embodiment is a hydroxyethylated starch known as "HES 150/1.0/1.3" for treatment of cancer when used in the described combination therapy. This is a hydroxyethylstarch with a mean molecular weight of 150 kDa, a molar substitution degree of 1.0 and a PDI of 1.3.

Another embodiment is a hydroxyethylated starch known as "Viastarch" with a mean molecular weight of: Mw 150--300 kDa,a molar substitution degree of MS 0.40--0.50, further characterized by Mw of lowest 10% fraction >=25 kDa, Mw of highest 10% fraction <=2000 kDa, which may also be referred to as "HES 180/0.45", for treatment of cancer when used in the described combination therapy.

Another embodiment is a hydroxyethylated starch known as "HES 200/0.5" for treatment of cancer when used in the described combination therapy. This is a hydroxyethylstarch with a mean molecular weight of 200 kDa, further characterized by a Mw 170-290, and of a molar substitution degree of 0.43 to 0.55. This HES may be further characterized by Mw of lowest 10% fraction >15 kDa, and Mw of highest 10% fraction <600 kDa.

Another embodiment is a hydroxyethylated starch known as "Pentastarch" with a mean molecular weight of: Mw 200-300 kDa, and a MS of 0.40-0.50; further characterized by Mw of lowest 10% fraction >=15 kDa, Mw of highest 10% fraction <=1500 kDa, which can be referred to as "HES 250/0.45", for treatment of cancer when used in the described combination therapy.

Another embodiment is a hydroxyethylated starch known as "HES 300/1.0/1.3" for treatment of cancer when used in the described combination therapy. This is a hydroxyethylstarch with a mean molecular weight of 250+/-17kDa (or 300 kDa according to Sommermeyer et al.), a molar substitution degree of 1.0+/-0.05 and a PDI of 1.3+/-0.1.

Another embodiment is a hydroxyethylated starch with a mean molecular weight of 300 kDa, a substitution degree Ds of below 0.4 as described in WO 00/48637, for treatment of cancer when used in the described combination therapy.

Another embodiment is a hydroxyethylated starch known as "HES 450/0.7" for treatment of cancer when used in the described combination therapy. This is a hydroxyethylstarch with a mean molecular weight of 450 kDa (Mw 400--500 kDa), which may be further specified by a Mw of lowest 10% fraction >=25 kDa, and a Mw of highest 10% fraction <=3000 kDa; and a molar substitution degree of 0.7 (MS 0.65--0.75).

Another embodiment is a hydroxyethylated starch with a mean molecular weight of 500 kDa according to the method referred to under Sommermeyer et al. and a molar substitution degree of 0.28 and a C2/C6 ratio of 8.7 described in and according to US patent 5,502,043 "Use of hydroxyethyl starch for improvement of microcirculation" to Weidler et al., in example 3, for the treatment of cancer when used in the described combination therapy.

Another embodiment is a hydroxyethylated starch with a mean molecular weight of 500 kDa and a molar substitution degree MS between 0.25 and 0.5 and a C2/C6 ratio of 2 to below 8 described in and according to European patent EP1732953B (claim 1), for the treatment of cancer when used in the described combination therapy.

Another embodiment is a hydroxyethylated starch with a mean molecular weight of 600 kDa and a molar substitution degree of 0.5 described in and according to European patent EP0402724B by Fresenius AG for the treatment of cancer when used in the described combination therapy.

Another embodiment is a hydroxyethylated starch known as "HES 700/0.5/2.5" for treatment of cancer when used in the described combination therapy. This is a hydroxyethylstarch with a mean molecular weight of 600+/-40 kDa (or 700 kDa according to Sommermeyer et al.), a molar substitution degree of 0.5+/-0.05 and a PDI of 2.5.

Another embodiment is a hydroxyethylated starch known as "Hetastarch", with a mean molecular weight of: Mw 550--800 kDa, MS 0.70--0.80, Mw of lowest 10% fraction >=13 kDa, Mw of highest 10% fraction <=4000 kDa; which can be described as "HES 700/0.7" for treatment of cancer when used in the described combination therapy.

Another embodiment is a hydroxyethylated starch known as "HES 700/0.7/2.0" for treatment of cancer when used in the described combination therapy. This is a hydroxyethylstarch with a mean molecular weight of 600+/-40 kDa (or 700 kDa according to Sommermeyer et al.), a molar substitution degree of 0.7+/-0.05and a PDI of 2.0.

Another embodiment is a hydroxyethylated starch known as "HES 700/1.0/1.5" for treatment of cancer when used in the described combination therapy. This is a hydroxyethylstarch with a mean molecular weight of 600+/-40 kDa

(or 700 kDa according to Sommermeyer et al.), a molar substitution degree of 1.0+/-0.05 and a PDI of 1.5.

Another embodiment is a hydroxyethylated starch known as "HES 700/1.3/1.5" for treatment of cancer when used in the described combination therapy. This is a hydroxyethylstarch with a mean molecular weight of 600+/-40 kDa (or 700 kDa according to Sommermeyer et al.), a molar substitution degree of 1.3+/-0.05and a PDI of 1.6+/-0.1.

Another embodiment is a hydroxyethylated starch known as "HES 60/1.3/1.3" for treatment of cancer when used in the described combination therapy. This is a hydroxyethylstarch with a mean molecular weight of 50+/-5 kDa (or 60 kDa according to Sommermeyer et al.), a molar substitution degree of 1.3+/-0.05 and a PDI of 1.3+/-0.1.

Another embodiment is a hydroxyethylated starch of a mean molecular weight Mw of 1000 kDa and a substitution degree Ds between 4 and 10 for, as described in US patent 6,680,305 for treatment of cancer when used in the described combination therapy.

Another embodiment is a hydroxyethylated starch known as "HES 70000", also referred to as "HES 70/0.55" with a mean molecular weight Mw of 60 - 80 kDa for treatment of cancer when used in the described combination therapy. Preferably it has a MS of 0.55-0.61. Preferably it has a PDI of 2.3 +/-0.1.

Another embodiment is a hydroxyethylated starch known of a mean molecular weight Mw of 70 kDa and a C2/C6 ratio between 2 to 8 as described in and according to A.N.Belder and B.Norman in Carbohydrate Research, Vol 10, 1969, p. 391-394 for treatment of cancer when used in the described combination therapy.

The method of treating a subject suffering from a tumor with a combination of HAS and one or more compounds selected from the group consisting of cytostatica, biologicals with anti-cancer activity and hormons with anti-cancer activity is one preferred embodiment of the invention. It is preferred that the method comprises a step of administering a therapeutically effective amount of said HAS to said subject and a step of administering one or more compounds selected from the group consisting of cytostatica, biologicals with anti-cancer activity and hormons with anti-cancer activity. The compound would be selected according to medical needs and clinical practice and routine, depending on the presented tumor. Preferably the dosage of the one or more compounds selected from the group consisting of cytostatica, biologicals with anti-cancer activity and hormons with anti-cancer activity is reduced in the method of treatment as compared to the administration of the compound alone.

Another preferred embodiment is the kit as described above wherein pharmaceutical composition comprising HAS is characterized as being therapeutically active in reducing the tumor growth rate. It is preferred that the HAS is the only therapeutically active ingredient in this pharmaceutical composition, that is part of the kit.

It is a preferred embodiment wherein the therapeutic activity of HAS results in an inhibitory effect on the proliferating activity of the tumor cells, wherein HAS reduces the proliferation rate of tumor cells. This is based on the observations which were made when the tumor tissue of treated and untreated mice was compared.

After the mice were sacrificed in treatment studies as described, for example, in Example 1, the tumor tissue was excised from the corpse and an analysis was performed to compare the effect of HES administered to tumor mice on intratumoral necrosis, number of mitotic figures, Ki67-index and CD31 staining intensity in a murine tumor model.

48 different tumor preparations have been analysed with a standard test based on the staining of KI positive cells. In addition a "CD31 staining" has been performed to determine the density of the tumor stroma. Due to technical difficulties only a limited set of samples could be analysed with the latter staining, but in all samples tested the density appeared to be similar.

In summary, comparison of tumor tissues of group A, from mice treated with saline only, with group V, from mice treated with Voluven 10%, revealed a decreased number of mitotic figures in group V, which is indicative for a decreased proliferative activity of tumor cells in group V. Group V tumors also contained slightly more necrosis than group A tumors. No obvious differences between group A and V were identified using a semiquantitative Ki67-index to analyze the percentage of proliferative tumor cells and a CD31-staining to analyze the density of intratumoral vessels.

Hence, it could be shown that HAS solutions have a direct effect on the proliferation rate of tumor cells, which is reduced in cells from HAS treated tumor tissue, whereas the treatment with HAS solutions does not affect normally proliferating cells in healthy tissues.

Therefore, without being bound to a theory, we assume that HAS treatment results in reducing the tumor growth rate by inhibition or deceleration of the cell proliferation rate of tumor cells, which is caused by a reduced number of tumor cells in mitosis (which refers to a reduced number of doubling tumor cells). The data indicate that the mitotic activity of the tumor cells is reduced.

It is an embodiment of the invention wherein HAS used in the combination therapy as described above is therapeutically active in reducing the tumor growth rate by reducing or inhibiting the proliferation rate or arresting the mitotic cycle of tumor cells or cells proliferating without physiological control.

It is another embodiment of the invention wherein HAS used in the combination therapy as described above is therapeutically active in reducing the tumour growth rate by arresting tumor cells in the mitotic cycle.

It could be shown that HAS solutions have a direct effect on the proliferation rate of tumor cells, whereas it can be assumed that the treatment with HAS solutions does not affect normally proliferating cells in healthy tissues.

In a preferred embodiment according to the invention the cancer is selected from the group of solid tumors

arising in solid tissues or organs.

In a preferred embodiment according to the invention the cancer is selected from the group consisting of biliary cancer, breast cancer, cervical cancer, colorectal cancer, gastrointestinal cancer, malignant melanoma, mesothelioma, myeloma, pancreatic cancer, prostate cancer, sarcoma, thyroid cancer, non-small cell lung cancer, and small cell lung cancer.

It is especially preferred that the cancer is selected from the group consisting of breast cancer, colorectal cancer, prostate cancer, and small cell and non-small cell lung cancer.

In one embodiment of the invention the cancer is breast cancer.

In one embodiment the cancer is selected from the group consisting of colorectal cancer, gastrointestinal cancer and kidney cancer.

In one embodiment the cancer is selected from the group consisting of small cell lung cancer and non-small cell lung cancer.

In one embodiment the cancer is selected from the group consisting of prostate cancer, and renal cancer.

In one embodiment the cancer is cervical cancer.

In one embodiment the cancer is selected from the group consisting of pancreatic cancer and biliary cancer.

In one embodiment the cancer is selected from the group consisting of sarcoma, mesothelioma and malignant melanoma.

In one embodiment according to the invention the tumor has been caused by a prostate cancer.

In another preferred embodiment according to the invention the tumor has been caused by a cancer selected from the group consisting of carcinoma. It is especially preferred that the tumor has been caused by a prostate carcinoma, a breast carcinoma, a lung carcinoma or a renal carcinoma.

It is especially preferred that said carcinoma are selected from the group consisting of skin, lung, colon, melanoma and ovarian carcinoma.

In another preferred embodiment of the invention the tumor is associated with a cancer selected from the group consisting of sarcoma.

In another preferred embodiment of the invention the tumor is associated with a cancer selected from the group consisting of cancer types that arise in the tissues of the brain and spinal cord.

It is especially preferred that the tumor which is treated with the combination therapy is a "fast growing tumor".

The term "fast growing tumor" is characterized as showing a tumor volume doubling time in a mouse model of less or equal to 4 days. The tumor volume doubling time (DT) is defined as the time interval (in days) required for a group of mice to reach a median RTV (relative tumor volume) of 200% of the initial tumor volume (normally from 100-200 $mm^3$). The tumor volume doubling time is an established parameter for the quantification of the tumor proliferation. Tumor volume doubling time for a given tumor may vary to some extent between experiments, which is why a tumor is classified a fast growing when it has a median doubling time (in a mouse model) beteen 1 and 4 (+/-0.4) days. A tumor is classified as slow growing when it has a median doubling time (in a mouse model) of more than 6 (+/-0.6) days. An example of a fast growing tumor is the tumor tissue characterized as LXFE 397.

It is especially preferred that the tumor is selected from the group of fast growing tumors of solid tumors arising in solid tissues or organs.

It is more preferred that the tumor is selected from the groups of fast growing carcinomas. It is even more preferred that the fast growing tumor is derived from a cancer selected from the group consisting of biliary cancer, breast cancer, cervical cancer, colorectal cancer, gastrointestinal cancer, malignant melanoma, mesothelioma, myeloma, pancreatic cancer, prostate cancer, renal cancer, sarcoma, thyroid cancer, non-small cell lung cancer, and small cell lung cancer.

In one embodiment the therapeutic effect of HAS given in combination with one or more compounds selected from the group consisting of cytostatica, biologicals with anti-cancer activity and hormons with anti-cancer activity, is further characterized as a treatment of cancer wherein significantly less toxic side effects for the treated patient are shown than when given one or more compounds selected from the group consisting of cytostatica, biologicals with anti-cancer activity and hormons with anti-cancer activity alone. The effect of the combination treatment according to the invention therefore is reducing the tumor growth rate, whilst not causing a severely decreased general health status.

Another preferred embodiment of the present invention pertains to the use of the hydroxyalkyl starch, or the pharmaceutical composition, according to the present invention for the manufacture of a medicament for the treatment of cancer, wherein the hydroxyalkyl starch is the therapeutically active ingredient, which is administered in combination with a pharmaceutical composition containing one or more compounds selected from the group consisting of cytostatica, biologicals with anti-cancer activity and hormons with anti-cancer activity. This combination may be administered within the same treatment day, within the same hour or with a treatment break in between, of up to several days or weeks.

The order of these two treatment steps may be either administering hydroxyalkylated starch prior to or after the administration of one or more compounds selected from the group consisting of cytostatica, biologicals with anti-cancer activity and hormons with anti-cancer activity or after.

Finally, the present invention also relates to a method of treatment of a subject in need thereof comprising of two steps, one being of the administering a therapeutically effective amount of hydroxyalkyl starch, as therapeutically active ingredient, to a subject in need thereof, resulting in stop or inhibition of the progression of cancer, preferably resulting in a reduced tumor size or reduced growth rate of the tumor and a second step of administering a one or more compounds selected from the group consisting of cytostatica, biologicals with anti-cancer activity and hormons with anti-cancer activity aiming to stop or inhibit the progression of cancer.

Treatment methods according to the invention may be targeted to all cancer types mentioned herein, and the HAS administered may comprise all types of HAS, and preferably HES disclosed herein.

The following especially preferred embodiments are described:

1. A hydroxyalkyl starch (HAS), as therapeutically active compound, administered in combination with one or more compounds selected from the group consisting of cytostatica, biologicals with anti-cancer activity and hormons with anti-cancer activity for treatment of cancer, characterized as a cancer comprising a growing tumor, wherein the administration of HAS results in reduced tumor growth rates, or a hydroxyalkyl starch (HAS), as therapeutically active compound, for reducing tumor growth rates, wherein the two therapeutically active compounds are not administered in one pharmaceutical composition, but preferably in two.

2. A hydroxyalkyl starch (HAS) according to embodiment 1 wherein the hydroxyalkyl starch comprises at least one structural unit according to the following formula (I)

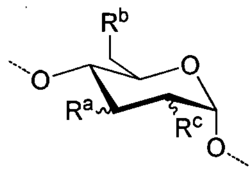

(I)

wherein $R^a$, $R^b$ and $R^c$ are independently of each other selected from the group consisting of-O-HAS", $-[O-(CR^wR^x)-(CR^yR^z)]_xOH$, $-[O-(CR^wR^x)-(CR^yR^z)]_y-XH$, wherein $R^w$, $R^x$, $R^y$ and $R^z$ are independently of each other selected from the group consisting of hydrogen and alkyl,
y is an integer in the range of from 0 to 20, preferably in the range of from 0 to 4, and x is an integer in the range of from 0 to 20, preferably in the range of from 0 to 4, and wherein at least one of $R^a$, $R^b$ and $R^c$ is $-[O-(CR^wR^x)-(CR^yR^z)]_y-XH$ and wherein X is selected from the group consisting of -S-, and -O-.

3. A hydroxyalkyl starch (HAS) according to embodiment 1, wherein the hydroxyalkyl starch has a mean molecular weight Mw between 20 and 1300 kDa.

4. A hydroxyalkyl starch (HAS) according to embodiment 1, wherein the hydroxyalkyl starch has a mean molecular weight Mw between 40 and 1300 kDa.

5. A hydroxyalkyl starch (HAS) according to embodiment 1, wherein the hydroxyalkyl starch has a mean molecular weight Mw between 65 and 1300 kDa.

6. A hydroxyalkyl starch (HAS) according to embodiment 1, wherein the hydroxyalkyl starch has a mean molecular weight Mw between 70 and 1200 kDa.

7. A hydroxyalkyl starch (HAS) according to embodiment 1, wherein the hydroxyalkyl starch has a mean molecular weight Mw between 75 and 800 kDa.

8. A hydroxyalkyl starch (HAS) according to embodiment 1, wherein the hydroxyalkyl starch has a mean molecular weight Mw between 90 and 800 kDa.

9. A hydroxyalkyl starch (HAS) according to any of the embodiments above, wherein the hydroxyalkyl starch has a mean molecular weight Mw between 100 and 700 kDa.

10. A hydroxyalkyl starch (HAS) according to any of the embodiments above, wherein the hydroxyalkyl starch has a mean molecular weight Mw between 100 and 110 kDa.

11. A hydroxyalkyl starch (HAS) according to any of the embodiments above, wherein the hydroxyalkyl starch has a mean molecular weight Mw above the renal threshold.

12. A hydroxyalkyl starch (HAS) according to embodiment 1 or 2, wherein the hydroxyalkyl starch has a mean molecular weight Mw below the renal threshold.

13. A hydroxyalkyl starch (HAS) according to any of the embodiments above, wherein the hydroxyalkyl starch has a molecular substitution MS between 0.1 and 1.5.

14. A hydroxyalkyl starch (HAS) according to any of the embodiments above, wherein the hydroxyalkyl starch has a molecular substitution MS between 0.1 and 1.3.

15. A hydroxyalkyl starch (HAS) according to any of the embodiments above, wherein the hydroxyalkyl starch has a molecular substitution MS between 0.1 and 1.1.

16. A hydroxyalkyl starch (HAS) according to any of the embodiments above, wherein the hydroxyalkyl starch has a molecular substitution MS between 0.1 and 0.9.

17. A hydroxyalkyl starch (HAS) according to any of the embodiments above, wherein the hydroxyalkyl starch has a molecular substitution MS between 0.3 and 0.8.

18. A hydroxyalkyl starch (HAS) according to any of the embodiments above, wherein the hydroxyalkyl starch has a molecular substitution MS between 0.3 and 0.7.

19. A hydroxyalkyl starch (HAS) according to embodiment 7 wherein the hydroxyalkyl starch has a mean molecular weight between 80 and 230 kDa and a molecular substitution MS between 0.3 and 0.6.

20. A hydroxyalkyl starch (HAS) according to embodiment 10 wherein the hydroxyalkyl starch has a mean molecular weight of 100 to 110 kDa and a molecular substitution MS between 0.3 and 0.5.

21. A hydroxyalkyl starch (HAS) according to embodiment 19 wherein the hydroxyalkyl starch has a mean molecular weight between 150 and 200 kDa and a molecular substitution MS between 0.4 and 0.5.

22. A hydroxyalkyl starch (HAS) according to embodiment 21 wherein the hydroxyalkyl starch has a mean molecular weight of 105 kDa and a molecular substitution MS of 0.42.

23. A hydroxyalkyl starch (HAS) according to embodiment 9 wherein the hydroxyalkyl starch has a mean molecular weight between 400 and 700 kDa and a molecular substitution MS between 0.6 and 0.8.

24. A hydroxyalkyl starch (HAS) according to any of the embodiments above wherein the hydroxyalkyl starch is hydroxethyl starch.

25. A hydroxyalkyl starch (HAS) according to any of the embodiments above wherein the HAS is provided as an aqueous solution and filled into a container suitable for clinical use. A preferred container for clinical use is a bottle or a bag or any other container made of glass or types of plastic materials that are authorized for the containment of drugs.

26. A hydroxyalkyl starch (HAS) according to any of the embodiments above wherein the treatment is characterized as inhibiting tumor growth or reducing tumor growth rates or tumor size, thereby achieving a dose reduction of one or more compounds selected from the group consisting of cytostatica, biologicals with anti-cancer activity and hormons with anti-cancer activity, compared to the dose given in a treatment without administration of HAS.

27. A hydroxyalkyl starch according to any of embodiments 1 to 26, wherein the cancer is selected from the group consisting of solid tumors, which are tumors arising in solid tissues.

29. A hydroxyalkyl starch according to any of embodiments 1 to 26, wherein the cancer is selected from the group consisting of cancer types arising in the skin or in tissues that line or cover internal organs, such as skin, lung, colon, pancreatic, ovarian, epithelial, squamous and basal cell carcinomas, melanomas, papillomas, and adenomas.

30. A hydroxyalkyl starch according to any of embodiments 1 to 26, wherein the cancer is selected from the group consisting of bone cancer, soft tissue cancer, osteosarcoma, synovialsarcoma, chondrosarcoma, liposarcoma, angiosarcoma, rhabdhosarcoma and fibrosarcoma.

31. A hydroxyalkyl starch according to any of embodiments 1 to 26, wherein the cancer is selected from the group consisting of brain and spinal cord tumors, gliomas, meningiomas, pituitary adenomas, vestibular schwannomas, primary CNS lymphomas, and neuroectodermal tumors.

32. A hydroxyalkyl starch according to any of embodiments 1 to 26, wherein the cancer is selected from the group consisting of biliary cancer, bladder cancer, breast cancer, cervical cancer, colorectal cancer, gastrointestinal cancer, malignant melanoma, mesothelioma, non-small cell lung cancer, pancreatic cancer, prostate cancer, sarcoma and small cell lung cancer.

33. A hydroxyalkyl starch according to embodiment 32, wherein the cancer is selected from the group consisting of bladder cancer, breast cancer, cervical cancer, colorectal cancer, prostate cancer, non-small cell lung cancer and small cell lung cancer.

34. A hydroxyalkyl starch according to embodiment 32, wherein the cancer is selected from the group consisting of breast cancer, prostate cancer, non-small cell lung cancer and small cell lung cancer.

35. A hydroxyalkyl starch according to any of the embodiments 27 to 34, wherein the cancer is characterized by a rapidly growing tumor type.

36. A hydroxyalkyl starch according to any of the embodiments 27 to 34, wherein the cancer is characterized as presenting a tumor type, characterized by a median doubling time of below 6 days, preferably of below or equal to 4 days, preferably this doubling time is determined in a mouse model suitable to determine cancer growth rates, more preferably in a mouse model as described in the examples.

37. A kit comprising a first pharmaceutical composition comprising a hydroxyalkyl starch (HAS) according to any of the embodiments 1 to 26, wherein HAS is the only therapeutically active component for the treatment of cancer, characterized as reducing the growth rate of tumors and a second pharmaceutical composition comprising one or more compounds selected from the group consisting of cytostatica, biologicals with anti-cancer activity and hormons

with anti-cancer activity.

38. A kit according to according to embodiment 36, wherein the pharmaceutical composition comprising a hydroxyalkyl starch (HAS) is , characterized as being contained in a suitable container, preferably made of glass or plastic materials, preferably HAS is provided therein as an aqueous solution.

39. A method of treating a subject suffering from cancer comprising administering a therapeutically effective amount of a hydroxyalkyl starch according to any of embodiments 1 to 26, in combination with one or more compounds selected from the group consisting of cytostatica, biologicals with anti-cancer activity and hormons with anti-cancer activity or a kit according to embodiment 36, thereby inhibiting progression of cancer, preferably by reducing the tumor growth rate, preferably whilst not reducing the proliferation rate of normal cells.

40. A method of treating a subject suffering from cancer according to claim 39 wherein the reducing of the tumor growth rate is achieved by reducing the proliferation rate of the tumor cells.

41. A method of treating a subject suffering from cancer according to claim 39 wherein the reducing of the tumor growth rate is achieved by arresting the tumor cells in the the mitotic cycle.

Description of figures:

Figure 1:

Figure **1** shows the development of the relative tumor volume of mice implanted with LXFE 397 tumor cells. Values on the Y-axis indicate the median relative tumor volume in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Capecitabine was administered to mice. The "♦" (black diamond) is used when a combination of Capecitabine and Voluven® 10 % (HES 130/0.4) was administered to mice, indicated as "Capecitabine + HES".

Figure 2:

Figure **2** shows the development of the relative body weight of mice implanted with LXFE 397 tumor cells. Values on the Y-axis indicate the median relative body weight in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Capecitabine was administered to mice. The "♦" (black diamond) is used when a combination of Capecitabine and Voluven® 10 % (HES 130/0.4) was administered to mice, indicated as "Capecitabine + HES".

Figure 3:

Figure **3** shows the development of the relative tumor volume of mice implanted with HNXF 1842 tumor cells. Values on the Y-axis indicate the median relative tumor volume in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Paclitaxel was administered to mice. The "♦" (black diamond) is used when a combination of Paclitaxel and Voluven® 10 % (HES 130/0.4) was administered to mice, indicated as "Paclitaxel + HES".

Figure 4:

Figure **4** shows the development of the median body weight of mice implanted with HNXF 1842 tumor cells. Values on the Y-axis indicate the median relative body weight in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Paclitaxel was administered to mice. The "♦" (black diamond) is used when a combination of Paclitaxel and Voluven® 10 % (HES 130/0.4) was administered to mice, indicated as "Paclitaxel + HES".

Figure 5:

Figure **5** shows the development of the relative tumor volume of mice implanted with RXF 1220 tumor cells. Values on the Y-axis indicate the median relative tumor volume in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Sunitnib was administered to mice. The "♦" (black diamond) is used when a combination of Sunitnib and Voluven® 10 % (HES 130/0.4) was administered to mice, indicated as "Sunitnib + HES".

Figure 6:

Figure 6 shows the development of the median body weight of mice implanted with RXF 1220 tumor cells. Values

on the Y-axis indicate the median relativebody weight in per cent. Values on the X-axis indicate the time in days after the start of treatment. The substances are indicated by the following symbols: the "▲" (black up-pointing triangle) is used when 0.9 % isotonic saline (NaCl) was administered to mice, indicated as "Control". The "■" (big black square) is used when Sunitnib was administered to mice. The "♦" (black diamond) is used when a combination of Sunitnib and Voluven® 10 % (HES 130/0.4) was administered to mice, indicated as "Sunitnib + HES".

**Examples:**

In these studies either the HES type commercially available under the name Voluven 10%, a "HES 130/04" as described in detail above was used, or a "HES 450/0.7", as described in detail in the specification with a Mw of 420 kDa measured according to European Pharmacopoeia, with a Dn/dc of 0.147 and a Ms of 0.7.

Animals and tumor implants were monitored daily until the maximum number of implants show clear signs of beginning solid tumor growth. At randomization of the animals, for example into different cages, the volume of growing tumors was initially determined. If not stated elsewhere, animals bearing at least one tumor of a volume of 50 - 250 $mm^3$, preferably 80 - 200 $mm^3$, were distributed in experimental groups according to the study protocol, considering a comparable median and mean of group tumor volume. The day of randomization was designated as day 0 of an experiment and was also the first day of dosing.

The tumor volume doubling time (DT), defined as the time interval (in days) required for a group of mice to reach a median RTV (relative tumor volume) of 200% of the initial tumor volume (normally from 100-200 $mm^3$), is routinely recorded in untreated or vehicle treated control groups of experiments and a median DT is calculated for characterization purposes.

Table 3:

| Tumormodel | Median doubling time (DT) | Growth characteristic |
|---|---|---|
| LXFE 397 | 1-4 | Fast growing |
| HNXF 1842 | 4-6 | Intermediately fast growing |
| RXF 1220 | 4-6 | Intermediately fast growing |

The tumor volume was determined by a two-dimensional measurement with callipers on the day of randomization (Day 0) and then once to twice weekly. Tumor volumes were calculated according to the following equation:

$$\text{Tumor Vol } [mm^3] = a\ [mm] \times b^2\ [mm^2] \times 0.5$$

where "a" is the largest diameter and "b" is the perpendicular diameter of the tumor representing an idealized ellipsoid.

The relative volume of an individual tumor on day X ($RTV_x$) was calculated by dividing the absolute volume [$mm^3$] of the respective tumor on day X ($T_x$) by the absolute volume of the same tumor on the day of randomization, i.e. on day 0 ($T_0$), multiplied by 100, as shown by the following equation:

$$RTV_x\ [\%] = \frac{T_x}{T_0} \times 100$$

Group median of RTVs were calculated, considering only the tumors of animals that were alive on the day in question. Group median RTVs are used for drawing tumor growth curves.

Starting on day 0, animals were weighed once to twice a week. Relative body weights (RBW) of individual animals are calculated by dividing the individual absolute body weight on day X ($BW_x$) by the individual body weight on the day of randomization, i.e. day 0 ($BW_0$), multiplied by 100, as shown by the following equation:

$$RBW_x\ [\%] = \frac{BW_x\ [g]}{BW_0\ [g]} \times 100$$

Group median of relative body weights were determined, considering only the weights of animals that were alive on the day in question .

The following termination criteria were applied to individual animals, irrespective of experimental status:

- tumor volume > 2000 $mm^3$ (unilateral)
- animals bearing ulcerating, skin-penetrating tumor
- body weight loss > 30%
- continued body weight loss > 20% for more than 7 days
- severe impairment of general condition (apathy, pain, markedly reduced feed and water intake, dyspnoea, abnormal habitus or behaviour)
- Whole groups were will be terminated if less than 3 animals were are left.

Example 1 LXFE 397:

Mice were either treated with Capecitabine at a dose of 100 mg/kg per orally (p.o.), with the plasma expander Voluven® 10% at a dose of 20 ml/kg bodyweight intravenously (i.v.), or with saline (20 ml/kg) i.v. at days 0, 4, 7, 11, 14, and 18. Tumor growth and body weight were determined over the course of the experiment.

Capecitabine (Xeloda®, Roche, lot: X9062B01) was stored at ambient temperature until use. Voluven® 10% (lot: 14EC3320) was obtained as ready to use product from Fresenius Kabi Deutschland GmbH and was stored at ambient temperature until use. Saline (0.9 % NaCl) was obtained from AlleMan Pharma.

The final solution of Capecitabine was prepared by dissolving 60 mg of compound in 6 ml of aqua ad iniectabilia (AlleMan Pharma) to obtain a solution with a concentration of 10 mg/ml immediately before injection. Saline and Voluven® were used in the original formulation. All solutions were prepared and injected under sterile conditions.

Tumor implants of the human tumor xenograft, LXFE 397/26N17 (Non-Small Cell Lung Cancer, histologically characterised as squamous cell carcinoma), derived from a primary tumor in the lung, poorly differentiated, were implanted subcutaneously (s.c.) into the left flank of immunodeficient female NMRI nu/nu mice under isoflurane anaesthesia. This tumor xenograft has a doubling time of 1-4 days .

Table 4

Treatment Groups

| Group | Mice [n] | Substances | Dose | Route | Days of dosing |
|---|---|---|---|---|---|
| **1** | 5 | Saline | 20 ml/kg | i.v. | 0,4,7,11,14,18 |
| **2** | 5 | Capecitabine | 100 mg/kg | p.o. | 0,4,7,11,14,18 |
| **3** | 5 | Voluven® 10% / Capecitabine | 20 ml/kg / 100 mg/kg | i.v. / p.o. | 0,4,7,11,14,18 |

The application volume was 20 ml/kg mouse body weight for saline (i.v.) and 100 mg/kg for Capecitabine (p.o.). Voluven® 10% and Capecitabine in combination were administered at a dose of 20 ml/kg (Voluven® 10%) and 100 mg/kg (Capecitabine) respectively. In case of the combination treatment Voluven® 10% was administered 1 hour prior to Capecitabine.

Results

The growth of the LXFE 397 tumor was monitored over a time period of 14 days and the results are shown in figure 1. In the control group the median relative tumor volume increased by approx. 19.0 fold compared to day 0. The reference drug Capecitabine moderately inhibited the tumor growth resulting in a slightly decelerated tumor growth, approx. 15.2 fold tumor volume increase over the 14 days. The combination of Capecitabine and Voluven® 10 % (indicated as "Capecitabine + HES") showed a much stronger inhibitory effect on the tumor growth as compared to the effect of the reference drug alone. The relative median tumor volume on day 14 was approx. 8.5 fold increased. The relative median body weight increased slightly over time wherein only the group which received Capecitabine only did not increase the body weight as steadily as the other two (figure 2).

Example 2: HNXF 1842

Mice were either treated with Paclitaxel at a dose of 10 mg/kg i.v., with a combination of Voluven® 10% (20 ml/kg i.v.) and Paclitaxel (10 mg/kg i.v.), or with saline (20 ml/kg) i.v. at days 0, 7, 14, and 21.Tumor growth and body weight were determined over the course of the experiment.

Tumor implants of the human tumor xenograft, HNXF 1842 (Head and Neck Cancer), originating from primary tumor in the oral cavity, moderately differentiated, and histologically characterised as a squamous cell carcinoma, were implanted subcutaneously (s.c.) into the left flank of immunodeficient female NMRI nu/nu mice under isoflurane anaesthesia. This tumor xenograft has a doubling time of 4-6 days.

Table 5:

Treatment Groups

| Group | Mice [n] | Substances | Dose | Route | Days of dosing |
|---|---|---|---|---|---|
| **1** | 5 | Saline | 20 ml/kg | i.v. | 0,7,14,21 |
| **2** | 5 | Paclitaxel | 10 mg/kg | i.v. | 0,7,14,21 |
| **3** | 5 | Voluven® 10%/ Paclitaxel | 20 ml/kg / 10 mg/kg | i.v. | 0,7,14,21 |

The application volume was 20 ml/kg mouse body weight for saline and Voluven 10% (i.v.) and 10 mg/kg for Paclitaxel (i.v.). Voluven 10% was administered 1 hour prior to Paclitaxel.

Results

The growth of the HNFX 1842 tumor was monitored over a time period of 18 days as shown in figure 3. In the control group the median relative tumor volume increased by approx. 12.1 fold compared to day 0. The reference drug Paclitaxel inhibited the tumor growth resulting in an approx. 7.1 fold tumor volume increase over the timespan of 18 days. The combination of Paclitaxel and Voluven® 10 % (indicated as "Paclitaxel + HES") showed a much stronger inhibitory effect on the tumor growth as compared to the effect of the reference drug alone. The relative median tumor volume on day 18 was approx. 4.0 fold increased. The relative median body weight increased slightly over time in all groups, wherein the mean body weight of the group receiving the Paclitaxel only had the lowest increase (figure 4).

Example 3: RXF 1220

Mice were either treated with Sunitinib at a dose of 40 mg/kg p.o., with a combination of Voluven® 10% (20 ml/kg i.v.) and Sunitinib (40 mg/kg p.o.), or with saline (20 ml/kg) i.v. at days 0-1 1.Tumor growth and body weight were determined over the course of the experiment.

Tumor implants of the human tumor xenograft, RXF 1220 (renal cancer), originating from metastases, and histologically characterized as hypernephroma, were implanted subcutaneously (s.c.) into the left flank of immunodeficient female NMRI nu/nu mice under isoflurane anaesthesia.

Table 5:

Treatment Groups

| Group | Mice [n] | Substances | Dose | Route | Days of dosing |
|---|---|---|---|---|---|
| **1** | 5 | Saline | 20 ml/kg | i.v. | 0-11 |
| **2** | 5 | Sunitinib | 40 mg/kg | p.o. | 0-11 |
| **3** | 5 | Voluven® 10% / Sunitinib | 20 ml/kg / 40 mg/kg | i.v. / p.o. | 0-11 |

The application volume was 20 ml/kg mouse body weight for saline and Voluven® 10% (i.v.) and 40 mg/kg for Sunitinib (p.o.). Voluven® 10% was administered 1 hour prior to Sunitinib.

Results

The growth of the RXF 1220 tumor was monitored over a time period of 27 days as shown in figure 5). In the control group the median relative tumor volume increased by approx. 15.7 fold compared to day 0. The reference drug Sunitinib inhibited the tumor growth resulting in an approx. 8.5 fold tumor volume increase over the timespan of 27 days. The combination of Sunitinib and Voluven® 10 % (indicated as "Sunitinib + HES") showed a stronger inhibitory effect on the tumor growth in the beginning of the experiment as compared to the reference drug alone. However, at day 27 the tumor volume increase was comparable to the reference drug with an approx. 8.2 fold increase. The relative median body weight increased slightly over time. The animals receiving the combination showed slightly higher body weights compared to the control group and the group receiving Sunitinib only (figure 6).

## Claims

1. A hydroxyethyl starch (HES), as therapeutically active compound for use in the treatment of cancer wherein the treatment is characterized as reducing the growth rate of tumors in a combination therapy with one or more compounds selected from the group consisting of cytostatica, biologicals with anti-cancer activity and hormons with anti-cancer activity wherein the combination therapy is the administration of a therapeutically effective amount of HES and a separate administration of a therapeutically effective amount of one or more compounds selected from the group consisting of cytostatica, biologicals with anti-cancer activity and hormons with anti-cancer activity.

2. A hydroxyethyl starch (HES), for use in the treatment of cancer according to claim 1, wherein HES is administered prior to administration of the one or more compounds selected from the group consisting of cytostatica, biologicals with anti-cancer activity and hormons with anti-cancer activity.

3. A hydroxyethyl starch (HES), for use in the treatment of cancer according to claim 1, wherein the one or more compounds selected from the group consisting of cytostatica, biologicals with anti-cancer activity and hormons with anti-cancer activity are administered prior to administration of HES.

4. A hydroxyethyl starch for use in the treatment of cancer according to any of the preceding claims, wherein the cancer is selected from the group of solid tumors, which are tumors arising in solid tissues.

5. A hydroxyethyl starch for use in the treatment of cancer according to any of the preceding claims, wherein the cancer is selected from the group of carcinoma.

6. A hydroxyethyl starch for use in the treatment of cancer according to any of the preceding claims, wherein the cancer is selected from the group consisting of cancer types arising in the skin or in tissues that line or cover internal organs, such as skin, lung, colon, pancreatic, ovarian, epithelial, squamous and basal cell carcinomas, melanomas, papillomas, and adenomas.

7. A hydroxyethyl starch for use in the treatment of cancer according to claim 5, wherein the cancer is selected from the group consisting of breast carcinoma, lung carcinoma, prostate carcinoma and renal carcinoma.

8. A hydroxyethyl starch for use in the treatment of cancer according to any of the claims 1 to 3, wherein the cancer is selected from the group consisting of bone cancer, soft tissue cancer, osteosarcoma, synovialsarcoma, chondrosarcoma, liposarcoma, angiosarcoma, rhabdhosarcoma and fibrosarcoma.

9. A hydroxyethyl starch for use in the treatment of cancer according to claims 1 to 3, wherein the cancer is selected from the group consisting of biliary cancer, bladder cancer, breast cancer, cervical cancer, colorectal cancer, gastrointestinal cancer, malignant melanoma, mesothelioma, non-small cell lung cancer, pancreatic cancer, prostate cancer, sarcoma and small cell lung cancer.

10. A hydroxyethyl starch (HES) for use in the treatment of cancer according to any of the preceding claims, wherein the hydroxyalkyl starch has a mean molecular weight (Mw) between 20 and 1300 kDa determined according to the calibration method described in the European Pharmacopeia 7.0, 01/2011:1785, p. 984, with a dn/dc value of 0.147+/-0.001.

11. A hydroxyethyl starch (HES) according to any of the preceding claims for use in the treatment of cancer according to any of claims 1 to 9, wherein the treatment is characterized as inhibiting a tumor cell from proliferating whilst not affecting a normally growing cell.

12. A hydroxyethyl starch (HES) according to any of the preceding claims for use in the treatment of cancer according to any of claims 1 to 9, wherein the treatment is characterized as arresting the mitotic cycle of the tumor cell.

13. A kit comprising a first pharmaceutical composition comprising a hydroxyethyl starch (HES) according to any of the preceding claims, wherein in said first composition HES is the only therapeutically active component for the treatment of cancer, characterized as reducing the growth rate of tumors; and a second pharmaceutical composition comprising one or more compounds selected from the group consisting of cytostatica, biologicals with anti-cancer activity and hormons with anti-cancer activity.

14. A kit according to claim 13, wherein the first pharmaceutical composition is characterized as being contained in a suitable container, preferably made of glass or plastic materials, and preferably HES is provided therein as an aqueous solution.

**Patentansprüche**

1. Hydroxyethylstärke (HES), als therapeutisch wirksame Verbindung zur Verwendung bei der Behandlung von Krebs, wobei die Behandlung als Reduktion der Tumorwachstumsrate in einer Kombinationstherapie mit einer oder mehreren aus der Gruppe bestehend aus Zytostatika, Biopharmazeutika mit krebshemmender Aktivität und Hormonen mit krebshemmender Aktivität ausgewählten Verbindungen gekennzeichnet ist, wobei es sich bei der Kombinationstherapie um die Verabreichung einer therapeutisch wirksamen Menge an HES und eine separate Verabreichung einer therapeutisch wirksamen Menge einer oder mehrerer aus der Gruppe bestehend aus Zytostatika, Biopharmazeutika mit krebshemmender Aktivität und Hormonen mit krebshemmender Aktivität ausgewählter Verbindungen handelt.

2. Hydroxyethylstärke (HES), zur Verwendung bei der Behandlung von Krebs nach Anspruch 1, wobei HES vor Verabreichung der einen oder mehreren aus der Gruppe bestehend aus Zytostatika, Biopharmazeutika mit krebshemmender Aktivität und Hormonen mit krebshemmender Aktivität ausgewählten Verbindungen verabreicht wird.

3. Hydroxyethylstärke (HES), zur Verwendung bei der Behandlung von Krebs nach Anspruch 1, wobei die eine oder mehreren aus der Gruppe bestehend aus Zytostatika, Biopharmazeutika mit krebshemmender Aktivität und Hormonen mit krebshemmender Aktivität ausgewählten Verbindungen vor Verabreichung von HES verabreicht werden.

4. Hydroxyethylstärke zur Verwendung bei der Behandlung von Krebs nach einem der vorhergehenden Ansprüche, wobei der Krebs aus der Gruppe der soliden Tumoren ausgewählt ist, bei denen es sich um Tumoren handelt, die in festen Geweben entstehen.

5. Hydroxyethylstärke zur Verwendung bei der Behandlung von Krebs nach einem der vorhergehenden Ansprüche, wobei der Krebs aus der Gruppe der Karzinome ausgewählt ist.

6. Hydroxyethylstärke zur Verwendung bei der Behandlung von Krebs nach einem der vorhergehenden Ansprüche, wobei der Krebs aus der Gruppe bestehend aus Krebsarten, die in der Haut oder in Geweben, die innere Organe säumen oder bedecken, entstehen, wie etwa Haut-, Lungen-, Kolon-, Pankreas-, Ovarial, Epithel-, Plattenepithel- und Basalzellkarzinome, Melanome, Papillome und Adenome, ausgewählt ist.

7. Hydroxyethylstärke zur Verwendung bei der Behandlung von Krebs nach Anspruch 5, wobei der Krebs aus der Gruppe bestehend aus Brustkarzinom, Lungenkarzinom, Prostatakarzinom und Nierenkarzinom ausgewählt ist.

8. Hydroxyethylstärke zur Verwendung bei der Behandlung von Krebs nach einem der Ansprüche 1 bis 3, wobei der Krebs aus der Gruppe bestehend aus Knochenkrebs, Weichteilkrebs, Osteosarkom, Synovialsarkom, Chondrosarkom, Liposarkom, Angiosarkom, Rhabdhosarkom und Fibrosarkom ausgewählt ist.

9. Hydroxyethylstärke zur Verwendung bei der Behandlung von Krebs nach einem der Ansprüche 1 bis 3, wobei der Krebs aus der Gruppe bestehend aus Krebs des Gallenwegsystems, Blasenkrebs, Brustkrebs, Gebärmutterhalskrebs, Kolorektalkarzinom, Krebs des Magen-Darm-Trakts, malignem Melanom, Mesotheliom, nichtkleinzelligem Lungenkrebs, Bauchspeicheldrüsenkrebs, Prostatakrebs, Sarkom und kleinzelligem Lungenkrebs ausgewählt ist.

10. Hydroxyethylstärke (HES) zur Verwendung bei der Behandlung von Krebs nach einem der vorhergehenden Ansprüche, wobei die Hydroxyethylstärke ein mittleres Molekulargewicht (Mw) zwischen 20 und 1300 kDa aufweist, bestimmt gemäß dem in der europäischen Pharmakopöe 7.0, 01/2011:1785, S. 984, beschriebenen Kalibrierungsverfahren bei einem dn/dc-Wert von 0,147+/-0,001.

11. Hydroxyethylstärke (HES) nach einem der vorhergehenden Ansprüche zur Verwendung bei der Behandlung von Krebs nach einem der Ansprüche 1 bis 9, wobei die Behandlung als Hemmen des Proliferierens einer Tumorzelle ohne Auswirkung auf eine normal wachsende Zelle gekennzeichnet ist.

12. Hydroxyethylstärke (HES) nach einem der vorhergehenden Ansprüche zur Verwendung bei der Behandlung von

Krebs nach einem der Ansprüche 1 bis 9, wobei die Behandlung als Anhalten des Mitosezyklus der Tumorzelle gekennzeichnet ist.

**13.** Kit, umfassend eine erste pharmazeutische Zusammensetzung, die eine Hydroxyethylstärke (HES) nach einem der vorhergehenden Ansprüche umfasst, wobei in der ersten Zusammensetzung HES die einzige therapeutisch wirksame Verbindung für die Behandlung von Krebs ist, die als Reduktion der Tumorwachstumsrate gekennzeichnet ist; und eine zweite pharmazeutische Zusammensetzung, die eine oder mehrere aus der Gruppe bestehend aus Zytostatika, Biopharmazeutika mit krebshemmender Aktivität und Hormonen mit krebshemmender Aktivität ausgewählte Verbindungen umfasst.

**14.** Kit nach Anspruch 13, wobei die erste pharmazeutische Zusammensetzung als enthalten in einem geeigneten Behälter, bevorzugt aus Glas oder Kunststoffmaterialien, gekennzeichnet ist und vorzugsweise HES darin als wässrige Lösung bereitgestellt ist.

## Revendications

**1.** Hydroxyéthyl-amidon (HES), en tant que composé thérapeutiquement actif pour utilisation dans le traitement d'un cancer, le traitement étant **caractérisé par** la réduction du taux de croissance de tumeurs dans une thérapie d'association avec un ou plusieurs composés choisis dans le groupe constitué d'agents cytostatiques, d'agents biologiques ayant une activité anticancéreuse et d'hormones ayant une activité anticancéreuse, la thérapie d'association étant l'administration d'une quantité thérapeutiquement efficace de HES et une administration séparée d'une quantité thérapeutiquement efficace d'un ou plusieurs composés choisis dans le groupe constitué d'agents cytostatiques, d'agents biologiques ayant une activité anticancéreuse et d'hormones ayant une activité anticancéreuse.

**2.** Hydroxyéthyl-amidon (HES), pour utilisation dans le traitement d'un cancer selon la revendication 1, HES étant administré avant l'administration des un ou plusieurs composés choisis dans le groupe constitué d'agents cytostatiques, d'agents biologiques ayant une activité anticancéreuse et d'hormones ayant une activité anticancéreuse.

**3.** Hydroxyéthyl-amidon (HES), pour utilisation dans le traitement d'un cancer selon la revendication 1, les un ou plusieurs composés choisis dans le groupe constitué d'agents cytostatiques, d'agents biologiques ayant une activité anticancéreuse et d'hormones ayant une activité anticancéreuse étant administrés avant l'administration de HES.

**4.** Hydroxyéthyl-amidon pour utilisation dans le traitement d'un cancer selon l'une quelconque des revendications précédentes, le cancer étant choisi dans le groupe des tumeurs solides, qui sont des tumeurs survenant dans des tissus solides.

**5.** Hydroxyéthyl-amidon pour utilisation dans le traitement d'un cancer selon l'une quelconque des revendications précédentes, le cancer étant choisi dans le groupe des carcinomes.

**6.** Hydroxyéthyl-amidon pour utilisation dans le traitement d'un cancer selon l'une quelconque des revendications précédentes, le cancer étant choisi dans le groupe constitué de types de cancer survenant dans la peau ou dans des tissus qui revêtent ou recouvrent des organes internes, tels que des cancers de la peau, du poumon, du côlon, du pancréas, de l'ovaire, épithélium, des carcinomes épidermoïdes et à cellules basales, des mélanomes, des papillomes et des adénomes.

**7.** Hydroxyéthyl-amidon pour utilisation dans le traitement d'un cancer selon la revendication 5, le cancer étant choisi dans le groupe constitué d'un carcinome du sein, un carcinome du poumon, un carcinome de la prostate et un carcinome rénal.

**8.** Hydroxyéthyl-amidon pour utilisation dans le traitement d'un cancer selon l'une quelconque des revendications 1 à 3, le cancer étant choisi dans le groupe constitué d'un cancer des os, un cancer des tissus mous, un ostéosarcome, un sarcome synovial, un chondrosarcome, un liposarcome, un angiosarcome, un rhabdomyosarcome et un fibrosarcome.

**9.** Hydroxyéthyl-amidon pour utilisation dans le traitement d'un cancer selon les revendications 1 à 3, le cancer étant choisi dans le groupe constitué d'un cancer des voies biliaires, un cancer de la vessie, un cancer du sein, un cancer du col de l'utérus, un cancer colorectal, un cancer gastro-intestinal, un mélanome malin, un mésothéliome, un cancer

du poumon non à petites cellules, un cancer du pancréas, un cancer de la prostate, un sarcome et un cancer du poumon à petites cellules.

**10.** Hydroxyéthyl-amidon (HES) pour utilisation dans le traitement d'un cancer selon l'une quelconque des revendications précédentes, dans lequel l'hydroxyalkyl-amidon a un poids moléculaire moyen (Mw) compris entre 20 et 1300 kDa déterminé selon le procédé d'étalonnage décrit dans la pharmacopée européenne 7.0, 01/2011:1785, p. 984, avec une valeur dn/dc de 0,147+/- 0,001.

**11.** Hydroxyéthyl-amidon (HES) selon l'une quelconque des revendications précédentes pour utilisation dans le traitement d'un cancer selon l'une quelconque des revendications 1 à 9, le traitement étant **caractérisé en ce qu'**il inhibe la prolifération d'une cellule tumorale, tout en n'ayant aucun effet sur une cellule croissant normalement.

**12.** Hydroxyéthyl-amidon (HES) selon l'une quelconque des revendications précédentes pour utilisation dans le traitement d'un cancer selon l'une quelconque des revendications 1 à 9, le traitement étant **caractérisé par** l'arrêt du cycle mitotique de la cellule tumorale.

**13.** Trousse comprenant une première composition pharmaceutique comprenant un hydroxyéthyl-amidon (HES) selon l'une quelconque des revendications précédentes, dans laquelle, dans ladite première composition HES est le seul composant thérapeutiquement actif pour le traitement du cancer, **caractérisé par** la réduction du taux de croissance de tumeurs ; et une deuxième composition pharmaceutique comprenant un ou plusieurs composés choisis dans le groupe constitué d'agents cytostatiques, d'agents biologiques ayant une activité anticancéreuse et d'hormones ayant une activité anticancéreuse.

**14.** Trousse selon la revendication 13, dans laquelle la première composition pharmaceutique est **caractérisée en ce qu'**elle est contenue dans un récipient adapté, de préférence constitué de verre ou de matières plastiques, et de préférence HES est disposé dans celui-ci sous la forme d'une solution aqueuse.

Figure 1

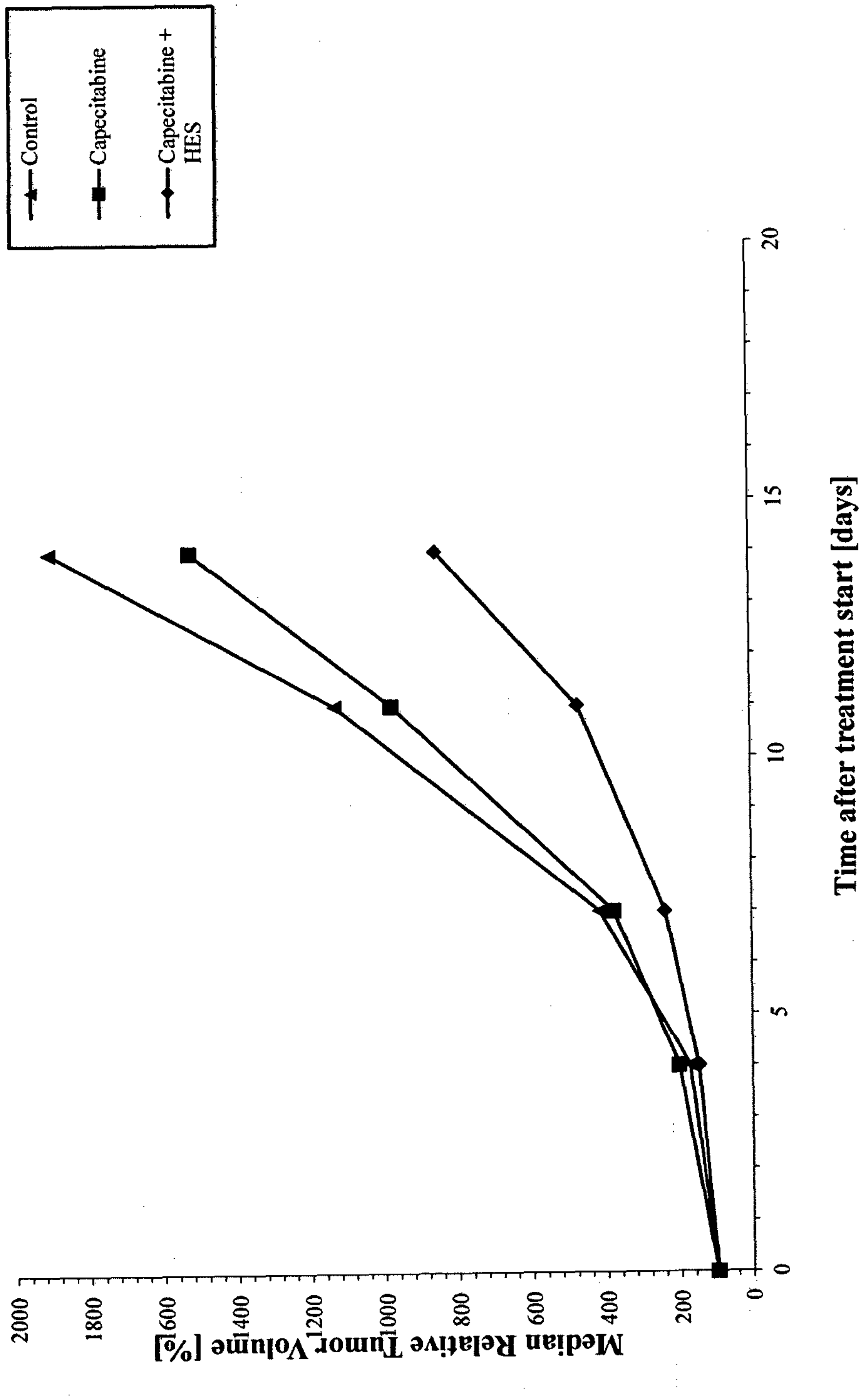
Control
Capecitabine
Capecitabine + HES
Median Relative Tumor Volume [%]
2000
1800
1600
1400
1200
1000
800
600
400
200
0
0
5
10
15
20
Time after treatment start [days]

Figure 2

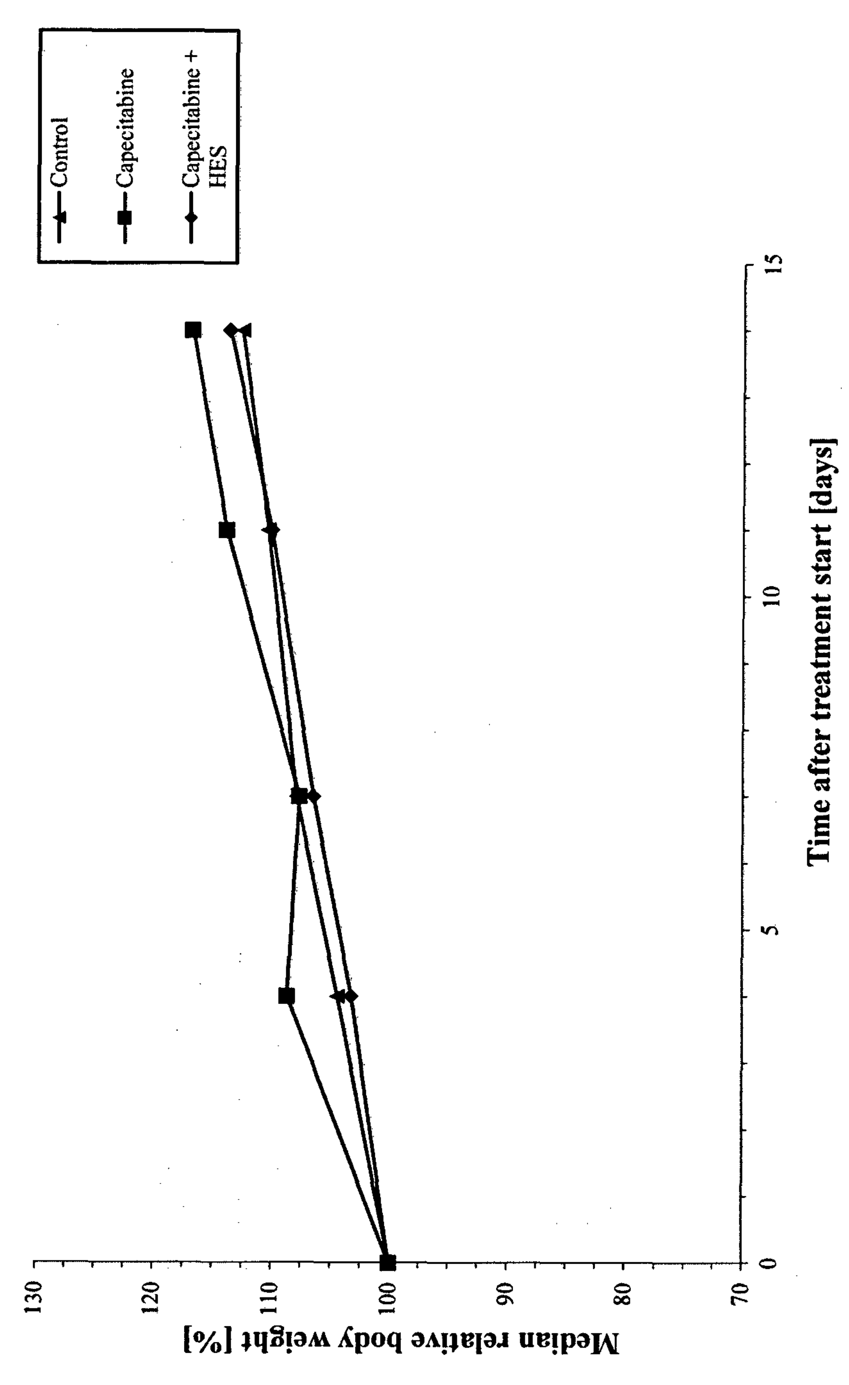
Control
Capecitabine
Capecitabine + HES
Median relative body weight [%]
130
120
110
100
90
80
70
0
5
10
15
Time after treatment start [days]

Figure 3

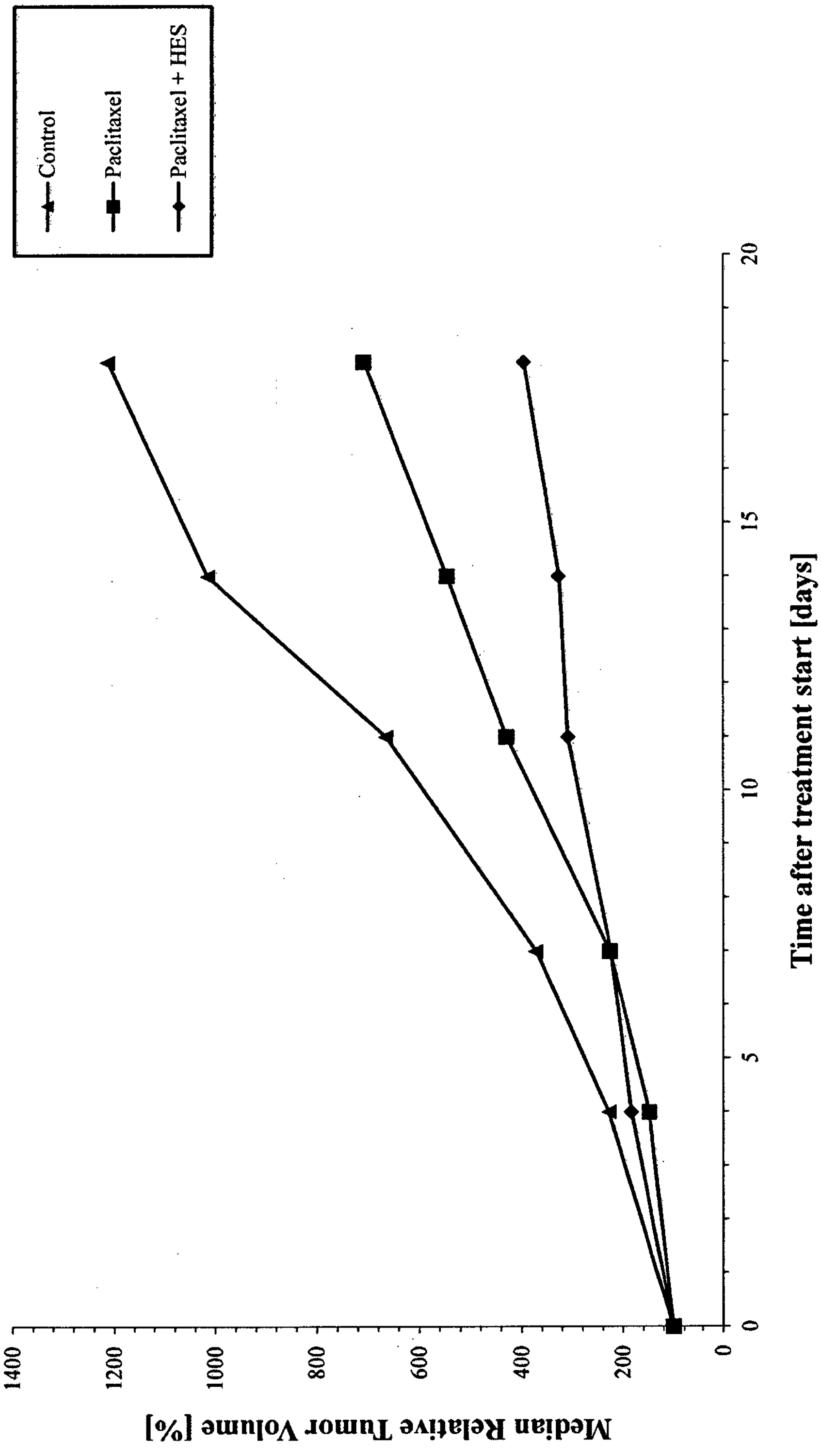
Control
Paclitaxel
Paclitaxel + HES
Median Relative Tumor Volume [%]
0
200
400
600
800
1000
1200
1400
0
5
10
15
20
Time after treatment start [days]

Figure 4

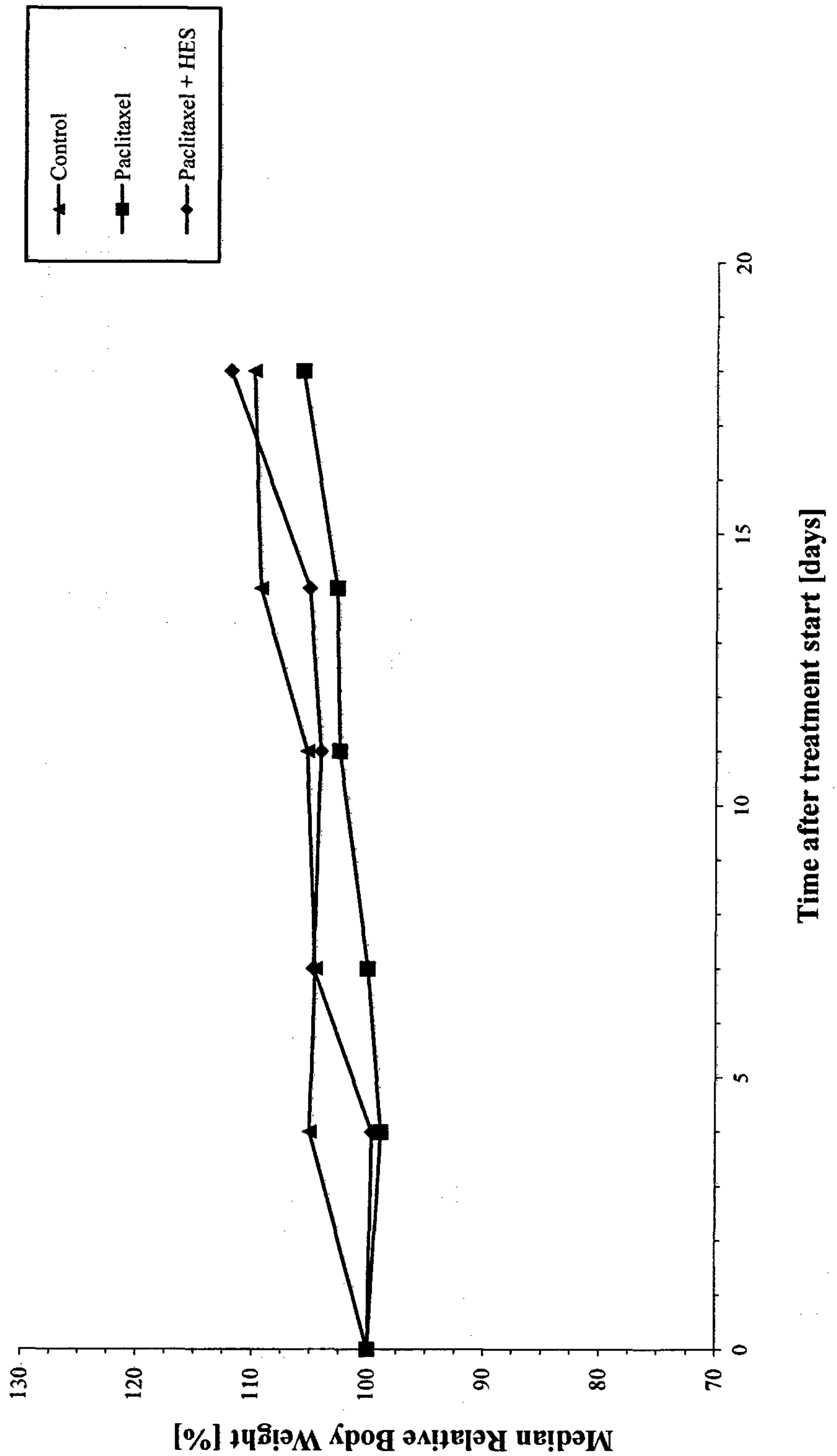
Control
Paclitaxel
Paclitaxel + HES
Median Relative Body Weight [%]
130
120
110
100
90
80
70
0
5
10
15
20
Time after treatment start [days]

Figure 5

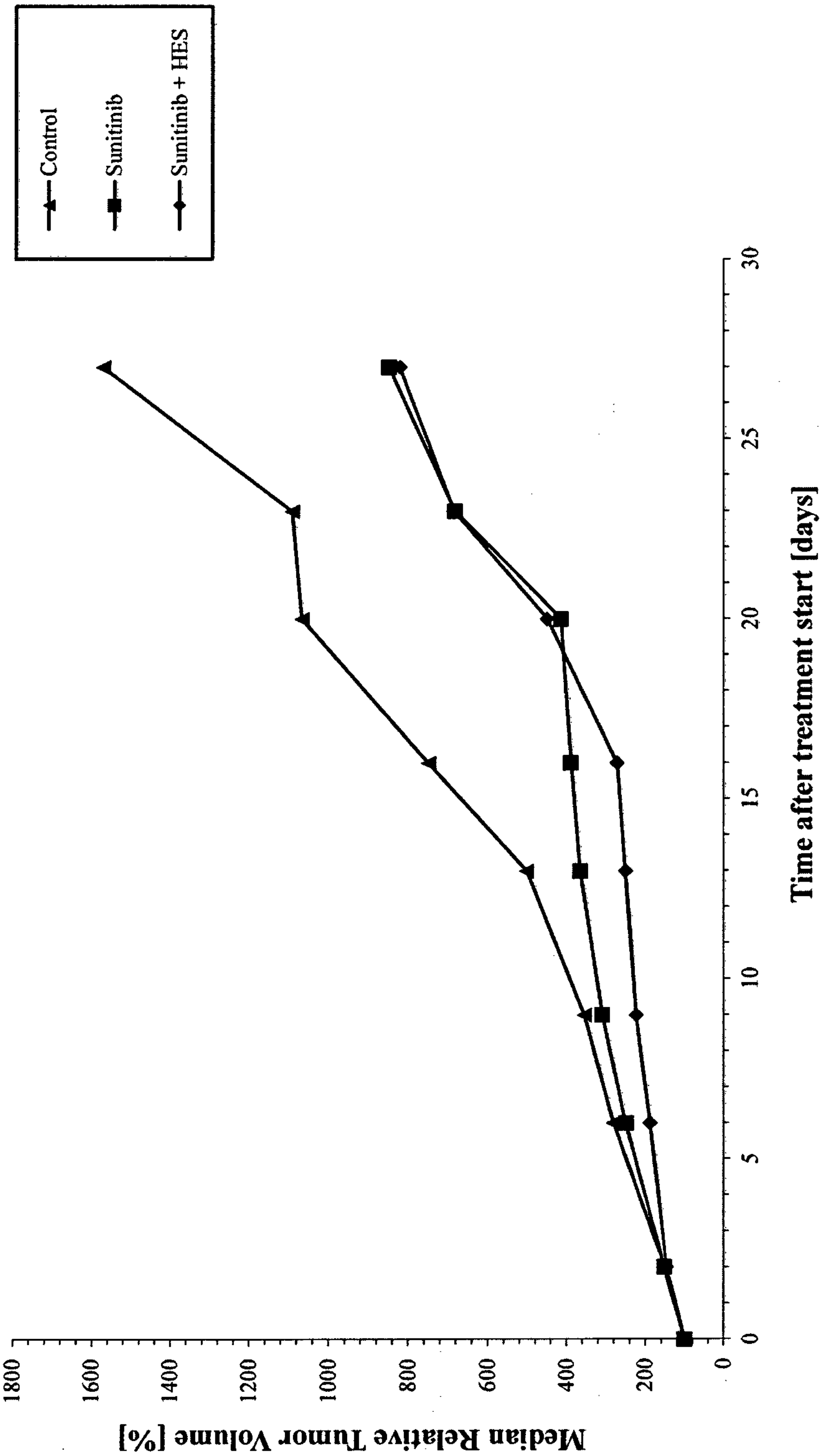
Median Relative Tumor Volume [%]
0
200
400
600
800
1000
1200
1400
1600
1800
Time after treatment start [days]
0
5
10
15
20
25
30
Control
Sunitinib
Sunitinib + HES

Figure 6

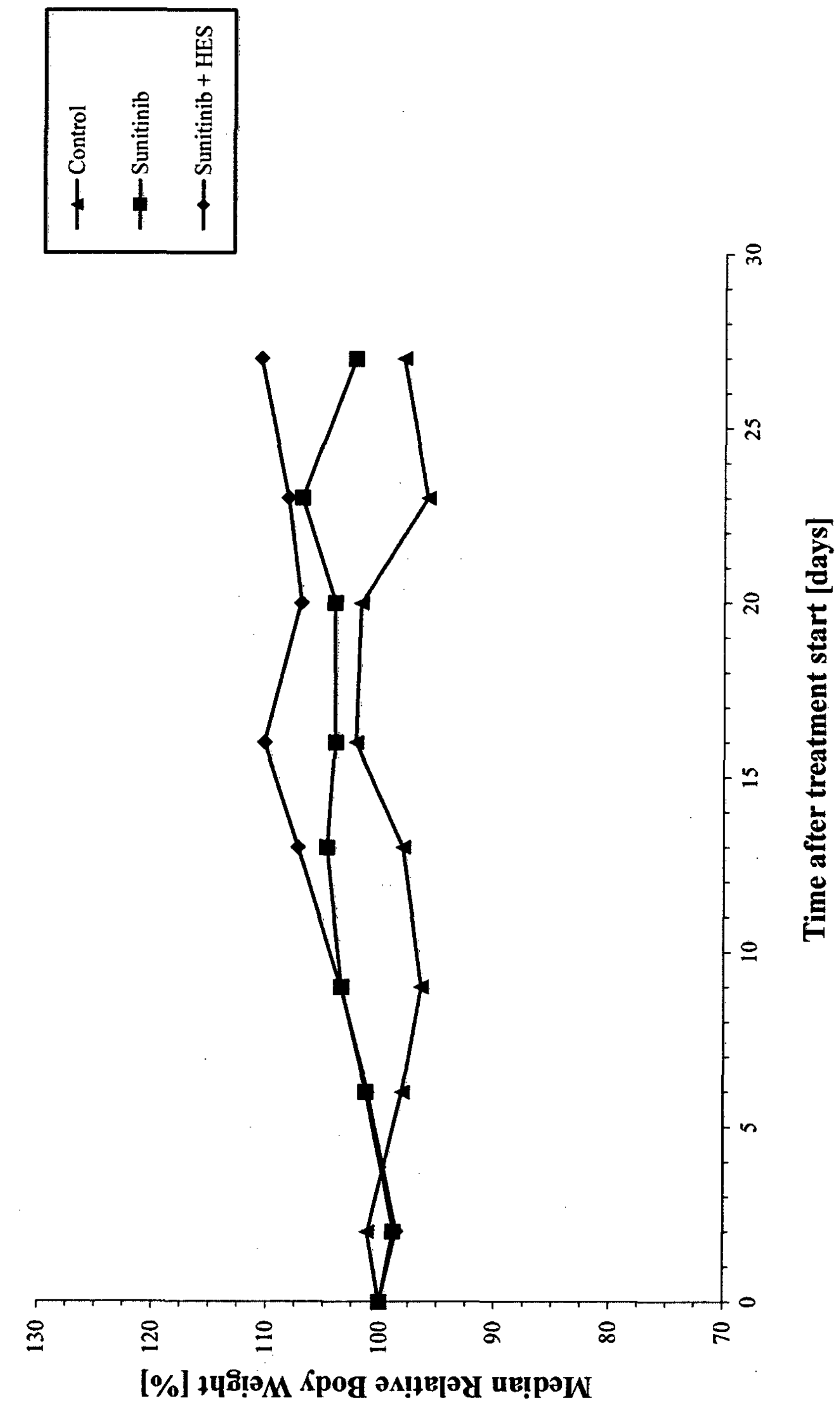
Control
Sunitinib
Sunitinib + HES
Median Relative Body Weight [%]
130
120
110
100
90
80
70
0
5
10
15
20
25
30
Time after treatment start [days]

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- DE 4023788, Schumann
- US 6207654 B, Zikria
- WO 9640108 A
- WO 03074088 A
- EP 2011003458 W
- WO 2012004005 PCT
- WO 0048637 A
- US 5502043 A
- EP 1732953 B
- EP 0402724 B
- US 6680305 B


### Non-patent literature cited in the description


- **SOMMERMEYER et al.** *Krankenhauspharmazie,* 1987, vol. 8 (8), 271-278
- **WEIDLER et al.** *Arzneimittelforschung/Drug Research,* 1991, vol. 41, 494-498
- **MOHAMED et al.** *European Journal of Surgical Oncology,* 2003, vol. 29, 261-265
- **KO et al.** Intravenous fluid therapy successfully prevents renal injury by gemcitabine in patients with pancreatic cancer. *Pancreas,* July 2011, vol. 40 (5), 784-6
- **SOMMERMEYER et al.** *Chromatographia,* 1988, vol. 25, 167-168
- **C. JUNGHEINRICH et al.** *Clin. Pharmacokin.,* 2005, vol. 44 (7), 681-699
- **J.-M. MISHLER IV.** Pharmacology of hydroxyethyl starches. Oxford Medical Publications, 2002, 1-30
- **W.-M. KULICKE et al.** *Starch,* 1993, vol. 45 (12), 445-450
- *European Pharmacopoeia 7.0,* January 2011, vol. 1785, 984
- **B. WITTGREN et al.** *Int. J. Polym. Anal. Charact.,* 2002, vol. 7 (1-2), 19-40
- **Y. M. LIU et al.** *Chin. Chem. Lett.,* 2002, vol. 13 (11), 1097-1099
- **W. BANKS et al.** *Br. J. Pharmac.,* 1973, vol. 47, 172-178
- **O. LARM et al.** *Starch,* 1981, vol. 33 (7), 240-244
- **SOMMERMEYER et al.** *Starch,* 1992, vol. 44 (6), 215-218
- **YING-CHE LEE et al.** *Anal. Chem.,* 1983, vol. 55, 334-338
- **K. L. HODGES et al.** *Anal. Chem.,* 1979, vol. 51, 2171
- **WAITZINGER et al.** *Clin. Drug Invest.,* 1998, vol. 16, 151-160
- **JUNGHEINRICH et al.** *Clin. Pharmacokinet.,* 2006, vol. 44 (7), 681-699
- **SOMMERMEYER et al.** *Krankenhauspharmazie,* 1987, vol. 8 (08), 271-278
- **SOMMERMEYER et al.** with a mean molecular weight of about 84 kDa (75-93 kDa). *European Pharmacopoeia 7.0,* January 2011, vol. 1785, 984
- **A.N.BELDER ; B.NORMAN.** *Carbohydrate Research,* 1969, vol. 10, 391-394